# EUROPÄISCHE PATENTANMELDUNG

(11) **EP 2 514 401 A2**
(43) Veröffentlichungstag der Anmeldung: **24.10.2012**
(21) Anmeldenummer: 12151599.3
(22) Anmeldetag: 18.01.2012
(51) Int. Cl.: A61K 8/37, A61K 8/42, A61K 8/67, A61Q 19/00

(54) **Kosmetische und dermatologische Zusammensetzungen gegen trockene Haut**

(30) Priorität: 20.01.2011 DE 102011002893
(71) Anmelder: Henkel AG & Co. KGaA, 40589 Düsseldorf (DE)
(72) Erfinder: Bähren, Annika, 41363 Jüchen (DE); Waldmann-Laue, Marianne, 40789 Monheim (DE); Knieps-Massong, Waltraud, 41352 Korschenbroich (DE); Holtkötter, Olaf, 50354 Hürth (DE)

(57) **Zusammenfassung**

Kombinationen aus 0,1 bis 0,5 Gew.-% mindestens eines Esters einer aliphatischen, mehrfach ungesättigten (C₁₆ bis C₃₀)-Carbonsäure; 0,05 bis 0,3 Gew.-% Panthenol; 0,01 bis 0,1 Tocopherylacetat; 0,5 bis 5 Gew.-% mindestens eines Harnstoff-Derivats der Formel (I) 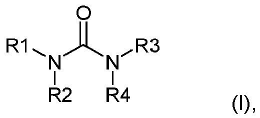 worin
die Reste R1, R2, R3 und R4 unabhängig voneinander für ein Wasserstoffatom, eine C₁-C₄-Alkylgruppe, eine C₂-C₆-Alkenylgruppe oder eine C₂-C₆-Hydroxyalkylgruppe mit mindestens einer Hydroxygruppe steht, mit der Maßgabe, dass mindestens einer der Reste R1, R2, R3 und R4 für eine C₂-C₆-Hydroxyalkylgruppe mit mindestens einer Hydroxygruppe steht,
besitzen sehr gute pflegende und hautnährende Eigenschaften und sind hervorragend gegen trockene und auch sehr trockene Haut wirksam.

## Beschreibung

Die vorliegende Erfindung betrifft topische kosmetische und dermatologische Zusammensetzungen, die in einem geeigneten kosmetischen oder dermatologischen Träger eine Kombination mindestens eines Esters einer aliphatischen, mehrfach ungesättigten (C₁₆ bis C₃₀)-Carbonsäure und mindestens eines speziellen Harnstoff-Derivats enthalten sowie deren Verwendung gegen trockene und sehr trockene Haut.

Das heute in der Fachwelt anerkannte Hautmodell von Elias beschreibt die Hornschicht als ZweiKomponenten-System, ähnlich einer Ziegelsteinmauer. In diesem Modell entsprechen die Hornzellen (Corneozyten) den Ziegelsteinen, die komplex zusammengesetzte Lipidmembran in den Interzellularräumen entspricht dem Mörtel. Dieses System stellt im Wesentlichen eine physikalische Barriere gegen hydrophile Substanzen dar, kann aber aufgrund seiner engen und mehrschichtigen Struktur gleichermaßen auch von lipophilen Substanzen nur schwer passiert werden. Die besondere Struktur der Hornschicht schützt einerseits die Haut und stabilisiert andererseits ihre eigene Flexibilität durch Bindung einer definierten Wassermenge.

Auch mechanische Belastungen, wie beispielsweise Druck-, Stoss- oder Scherkräfte, können in erstaunlichem Masse durch die Hornschicht allein oder im Verbund mit den tieferen Hautschichten abgefangen werden. Größere Druck-, Dreh- oder Scherkräfte werden über die Verzahnung der Epidermis mit dem Corium an tiefere Hautschichten weitergegeben. Die Regulation des Wasser-und Feuchtigkeitsgehaltes ist eine der wichtigsten Funktionen der epidermalen Lipidmembran. Allerdings hat sie nicht nur eine Barrierewirkung gegen externe chemische und physikalische Einflüsse, sondern trägt auch zum Zusammenhalt der Hornschicht bei.

Die Lipide der Hornschicht bestehen im Wesentlichen aus Ceramiden, freien Fettsäuren, Cholesterin sowie Cholesterinsulfat und sind über die gesamte Hornschicht verteilt. Die Zusammensetzung dieser Lipide ist für die intakte Funktion der epidermalen Barriere und damit für die Wasserundurchlässigkeit der Haut von entscheidender Bedeutung.

Bereits bei einer Reinigung der Haut mit Hilfe eines einfachen Wasserbades - ohne Zusatz von Tensiden- kommt es zunächst zu einer Quellung der Hornschicht der Haut. Der Grad dieser Quellung hängt u. a. von der Dauer des Bads und dessen Temperatur ab. Gleichzeitig werden wasserlösliche Stoffe ab- bzw. ausgewaschen, wie z. B. wasserlösliche Schmutzbestandteile, aber auch hauteigene Stoffe, die für das Wasserbindungsvermögen der Hornschicht verantwortlich sind. Durch hauteigene oberflächenaktive Stoffe werden außerdem auch Hautfette in gewissem Ausmaß gelöst und ausgewaschen. Dies bedingt nach anfänglicher Quellung eine nachfolgende Austrocknung der Haut, die durch waschaktive Zusätze noch deutlich verstärkt werden kann.

Bei gesunder Haut sind diese Vorgänge im allgemeinen belanglos, da die Schutzmechanismen der Haut solche leichten Störungen der oberen Hautschichten ohne weiteres kompensieren können. Aber bereits im Fall nicht-pathologischer Abweichungen vom Normalstatus, z. B. durch umweltbedingte Abnutzungsschäden bzw. Irritationen, Lichtschäden, Altershaut usw., ist der Schutzmechanismus an der Hautoberfläche gestört. Bei alter Haut beispielsweise erfolgt die regenerative Erneuerung verlangsamt, wobei insbesondere das Wasserbindungsvermögen der Hornschicht nachlässt. Sie wird deshalb inflexibel, trocken und rissig ("physiologisch" trockene Haut). Ein Barriereschaden ist die Folge. Die Haut wird anfällig für negative Umwelteinflüsse wie die Invasion von Mikroorganismen, Toxinen und Allergenen. Als Folge kann es sogar zu toxischen oder allergischen Hautreaktionen kommen. Trockene Haut weist einen Mangel an Barrierelipiden (Ceramide, Cholesterol und freie Fettsäuren) in der Hornschicht auf. Die verminderte Integrität und Reparaturleistung der Hautbarriere führt zu einem gesteigerten transepidermalen Wasserverlust. Die Haut wird rau, trocken, schuppig und neigt zu Spannungsgefühlen sowie Juckreiz.

Die verhornte Epidermis bildet den Schutzschild der Haut. Damit diese Funktion optimal erfüllt wird, müssen die Hautzellen (Keratinozyten) den Prozess der so genannten epidermalen Differenzierung durchlaufen. Nach der Teilung der Zellen in der Basalschicht wandern die Keratinozyten zur Hautoberfläche und machen dabei eine Reihe von Veränderungen durch, bis sie als tote, flache, kernlose Komeozyten die Hornschicht (stratum comeum) bilden und schließlich abgeschuppt werden. Während der epidermalen Differenzierung werden verschiedene Proteine mit spezifischen Funktionen ausgebildet. Hierzu zählen unter anderem Keratine, Involucrin, Filaggrin und Transglutaminase. Zur optimalen Ausbildung der Epidermis und der Hornschicht ist es notwendig, dass diese Proteine koordiniert und in ausreichender Menge gebildet werden.

Das Adenosintriphosphat (ATP) ist ein zentrales Molekül des Energiehaushalts lebender Zellen, da es die Energiequelle vieler zellulärer Reaktionen darstellt. Der intrazelluläre ATP-Gehalt ist ein sensitiver Marker für die Vitalität und Aktivität von Zellen, da eine erhöhte zelluläre Aktivität, z. B. während der Proliferation (Zellvermehrung), mit einem erhöhten Bedarf und Verbrauch von ATP korreliert.

Bei bestimmten Hautkrankheiten wie Ichthyosis oder atopischer Dermatitis sowie bei Altershaut wurden Störungen im Differenzierungsprozess festgestellt und mit den entsprechenden klinischen Hautveränderungen in Verbindung gebracht. Hauterkrankungen können z. B. auf eine Verminderung des Filaggringehalts zurückzuführen sein. Auch in der Altershaut sind viele natürliche Komponenten vermindert, wie beispielsweise der Gehalt an Collagen, Elastin, bestimmten Ceramiden und freien Aminosäuren.

Die Altershaut wird kosmetisch in erster Linie mit Vitamin A-Derivaten oder Hydroxysäuren behandelt, die über eine Stimulation der Proliferation der Basalzellen in der Epidermis zu einer Verdickung der Epidermis und damit Glättung der Haut führen. Neuere Ansätze bestehen darin, die Proteine, die in der trockenen oder der Altershaut fehlen oder vermindert vorkommen, gezielt zu substituieren bzw. indirekt in die Stoffwechselprozesse einzugreifen, die bei trockener Haut oder mit zunehmendem Alter gestört sind, um diese zu normalisieren. Als Beispiel sei hier die Stimulation der Collagensynthese mit dem Zweck der Faltenreduzierung angeführt. Weiterhin werden beispielsweise Laminin, Substanzen zur Verlängerung der Lebenszeit von Hautzellen und bestimmte Extrakte zur Stimulierung der epidermalen Differenzierung eingesetzt. Hierbei handelt es sich teilweise jedoch um pharmakologisch wirksame Substanzen mit hohem Nebenwirkungspotenzial.

Es besteht also ein ständiger Bedarf an neuen, nachweislich bioaktiven Komponenten mit Wirkung auf die biochemischen Prozesse, die zu trockener Haut und zu Altershaut führen. Die Erfindung geht daher von der Aufgabenstellung aus, ein bioaktives Substanzgemisch zur Verfügung zu stellen, das die Synthese von Adenosintriphosphat (ATP) in den Hautzellen stimuliert. Die so bereitgestellte Energie steht der Haut für erhöhte Stoffwechselleistungen, z. B. die Hauterneuerung, oder Reparaturprozesse, z. B. bei UV-Schäden, zur Verfügung. Die Substanz soll außerdem gezielt die Synthese der Proteine, die in der trockenen oder in der Altershaut vermindert sind, stimulieren. Letztlich wird so das Hautbild bei trockener Haut oder Altershaut von innen her verbessert durch Regulation eines relevanten biochemischen Prozesses. Ebenso wird die Schutzfunktion der Haut wiederhergestellt.

Es wurde nun gefunden, dass eine Kombination mindestens eines Esters aus der Gruppe der Linolsäure-, Linolensäure- und Arachidonsäureester, Panthenol, Tocopherylacetat und mindestens eines speziellen Harnstoff-Derivats sehr gute pflegende und hautnährende Eigenschaften besitzt und hervorragend gegen trockene und auch sehr trockene Haut wirksam ist.

Gegenstand der vorliegenden Erfindung sind in einer ersten Ausführungsform kosmetische oder dermatologische Mittel, enthaltend - bezogen auf ihr Gewicht -
a) 0,1 bis 0,5 Gew.-% mindestens eines Esters einer aliphatischen, mehrfach ungesättigten (C₁₆ bis C₃₀)-Carbonsäure (insbesondere ausgewählt aus einem oder mehrerer Ester der Gruppe, die gebildet wird aus Linolsäureestern, Linolensäurestern und Arachidonsäureestern);
b) 0,05 bis 0,3 Gew.-% Panthenol;
c) 0,01 bis 0,1 Tocopherylacetat;
d) 0,5 bis 5 Gew.-% mindestens eines Harnstoff-Derivats der Formel (I) 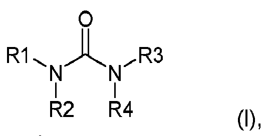
worin
die Reste R1, R2, R3 und R4 unabhängig voneinander für ein Wasserstoffatom, eine C₁-C₄-Alkylgruppe, eine C₂-C₆-Alkenylgruppe oder eine C₂-C₆-Hydroxyalkylgruppe mit mindestens einer Hydroxygruppe steht, mit der Maßgabe, dass mindestens einer der Reste R1, R2, R3 und R4 für eine C₂-C₆-Hydroxyalkylgruppe mit mindestens einer Hydroxygruppe steht.

Die erfindungsgemäßen Zusammensetzungen können in verschiedenen Angebotsformen konfektioniert werden, beispielsweise als (ggf. öl- und fettfreies) Gel, als Creme, in Stiftform, als flüssige oder gelförmige Roll-on-Applikation, als getränktes flexibles Substrat (Pad), aber auch als Puder oder Spray.

Erfindungsgemäße Mittel können in fester, halbfester, flüssiger, disperser, emulgierter, suspendierter, oder gelförmiger Form vorliegen. Weiterhin können die erfindungsgemäßen Mittel als Aerosol konfektioniert sein, das heißt, sie sind in einem Druckbehälter verpackt, aus dem sie mit Hilfe eines Treibmittels versprüht werden können. Weiterhin können die erfindungsgemäßen Mittel als treibgasfreies Pumpspray versprüht werden.

Als ersten wesentlichen Inhaltsstoff enthalten die erfindungsgemäßen Mittel bezogen auf ihr Gewicht 0,1 bis 0,5 Gew.-% mindestens eines Esters einer aliphatischen, mehrfach ungesättigten (C₁₆ bis C₃₀)-Carbonsäure. Bevorzugt werden diese Ester ausgewählt aus einer oder mehrerer Verbindungen der Gruppe, die gebildet wird aus Linolsäureestern, Linolensäurestern und Arachidonsäureestern.

Eine aliphatische, mehrfach ungesättigte (C₁₆ bis C₃₀)-Carbonsäure besitzt mindestens zwei ungesättigte Bindungen. Eine ungesättigte Bindung ist eine Doppelbindung oder eine Dreifachbindung jeweils zwischen zwei Kohlenstoffatomen.

Diese Verbindungen sind Ester (z.B. der Säuren Linolsäure (9Z,12Z-Octadecadiensäure), alpha-Linolensäure (9Z,12Z,15Z-Octadecatriensäure), Arachidonsäure (5Z,8Z,11Z,14Z-Eicosatetraensäure)) mit Alkoholen.

Bevorzugte Alkohole, mit denen die aliphatischen, mehrfach ungesättigten (C₁₆ bis C₃₀)-Carbonsäuren bzw. deren bevorzugte Vertreter verestert sein können sind Methanol, Ethanol, n-Propanol, Isopropanol, n-Butanol, Isobutanol, sec-Butanol und tert-Butanol sowie die mehrwertigen Alkohole Glycol, Glycerin, Pentaerythrit, Sorbit.

Besonders bevorzugte erfindungsgemäße Mittel enthalten als Ester einer aliphatischen, mehrfach ungesättigten (C₁₆ bis C₃₀)-Carbonsäure einen oder mehrere Glycerinester der zuvor genannten Säuren. Für die erfindungsgemäßen Mittel eignet sich bevorzugt der Einsatz mindestens eines Esters der Formel (E1) 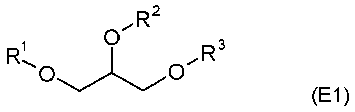 worin
R¹, R² und R³ stehen unabhängig voneinander für eine aliphatische, mehrfach ungesättigte (C₁₆ bis C₃₀)-Acylgruppe, insbesondere für dieselbe aliphatische, mehrfach ungesättigte (C₁₆ bis C₃₀)-Acylgruppe. R¹, R² und R³ werden ganz besonders bevorzugt ausgewählt aus Arachidonoyl (5Z,8Z,11Z,14Z-Eicosatetraenoyl), Linolenoyl (9Z,12Z,15Z-Octadecatrienoyl), Linoloyl (9Z,12Z-Octadecadienoyl).

Am bevorzugtesten eignet sich mindestens ein Ester, ausgewählt aus der Gruppe, die gebildet wird aus Glyceryltrilinolenat (z.B. CAS-Nr.: 14465-68-0) (INCI-Bezeichnung: Trilinolenin), Glyceryltrilinoleat (z.B. CAS Registry Nummer: 537-40-6) (INCI-Bezeichnung: Trilinolein), Triarachidoylglyzerin (z.B. CAS Registry Nummer: 620-64-4) (INCI-Bezeichnung: Triarachidin), oder Gemischen aus diesen Estern.

Unabhängig vom eingesetzten Ester sind erfindungsgemäße kosmetische oder dermatologische Mittel bevorzugt, die als Ester aliphatischer, mehrfach ungesättigter (C₁₆ bis C₃₀)-Carbonsäuren 0,15 bis 0,45 Gew.-%, vorzugsweise 0,2 bis 0,4 Gew.-%, weiter bevorzugt 0,25 bis 0,35 Gew.-% und insbesondere 0,28 bis 0,32 Gew.-% mindestens eines Esters enthalten, ausgewählt aus einer oder mehrerer Verbindungen der Gruppe, die gebildet wird aus Linolsäureestern, Linolensäureester und Arachidonsäureester. Weiter bevorzugt sind erfindungsgemäße kosmetische oder dermatologische Mittel, die 0,15 bis 0,45 Gew.-%, vorzugsweise 0,2 bis 0,4 Gew.-%, weiter bevorzugt 0,25 bis 0,35 Gew.-% und insbesondere 0,28 bis 0,32 Gew.-% mindestens eines Esters aus der Gruppe, die gebildet wird aus Linolsäure-Methylester, LinolsäureEthylester, Linolsäure-Propylester, Linolsäure-Isopropylester, Linolsäure-n-Butylester, Linolsäure-Isobutylester, Linolsäure-sec-Butylester, Linolsäure-tert-Butylester, Linolsäure-Glycolester, Linolsäure-Glycerinester, Linolsäure-Pentaerythritester, Linolsäure-Sorbitester,Linolensäure-Methylester, Linolensäure-Ethylester, Linolensäure-Propylester, Linolensäure-Isopropylester, Linolensäure-n-Butylester, Linolensäure-Isobutylester, Linolensäure-sec-Butylester, Linolensäuretert-Butylester, Linolensäure-Glycolester, Linolensäure-Glycerinester, Linolensäure-Pentaerythritester, Linolensäure-Sorbitester,Arachidonsäure-Methylester, ArachidonsäureEthylester, Arachidonsäure-Propylester, Arachidonsäure-Isopropylester, Arachidonsäure-n-Butylester, Arachidonsäure-Isobutylester, Arachidonsäure-sec-Butylester, Arachidonsäure-tert-Butylester, Arachidonsäure-Glycolester, Arachidonsäure-Glycerinester, Arachidonsäure-Pentaerythritester, Arachidonsäure-Sorbitester, enthalten.

Ganz besonders bevorzugte erfindungsgemäße Mittel enthalten Ethylester der genannten Säuren. Dementsprechend sind besonders bevorzugte erfindungsgemäße kosmetische oder dermatologische Mittel dadurch gekennzeichnet, dass sie 0,15 bis 0,45 Gew.-%, vorzugsweise 0,2 bis 0,4 Gew.-%, weiter bevorzugt 0,25 bis 0,35 Gew.-% und insbesondere 0,28 bis 0,32 Gew.-% mindestens eines Esters aus der Gruppe, die gebildet wird aus Linolsäureethylester, Linolensäureethylester und Arachidonsäureethylester, enthalten.

Ganz besonders bevorzugte Ester einer aliphatischen, mehrfach ungesättigten (C₁₆ bis C₃₀)-Carbonsäure sind solche mit Glycerin als Alkohol. Dementsprechend sind besonders bevorzugte erfindungsgemäße kosmetische oder dermatologische Mittel dadurch gekennzeichnet, daß sie 0,15 bis 0,45 Gew.-%, vorzugsweise 0,2 bis 0,4 Gew.-%, weiter bevorzugt 0,25 bis 0,35 Gew.-% und insbesondere 0,28 bis 0,32 Gew.-% mindestens eines Esters aus der Gruppe, die gebildet wird aus Linolsäureglycerinester, Linolensäureglycerinester und Arachidonsäureglycerinester, (insbesondere ausgewählt aus mindestens einem Ester der Formel (E1) oder aus deren bevorzugten Vertretern) enthalten.

Dementsprechend sind ganz besonders bevorzugte erfindungsgemäße kosmetische oder dermatologische Mittel dadurch gekennzeichnet, daß sie 0,15 bis 0,45 Gew.-%, vorzugsweise 0,2 bis 0,4 Gew.-%, weiter bevorzugt 0,25 bis 0,35 Gew.-% und insbesondere 0,28 bis 0,32 Gew.-% mindestens eines Esters aus der Gruppe, die gebildet wird aus Glyceryltrilinolenat (z.B. CAS-Nr.: 14465-68-0) (INCI-Bezeichnung: Trilinolenin), Glyceryltrilinoleat (z.B. CAS Registry Nummer: 537-40-6) (INCI-Bezeichnung: Trilinolein), Triarachidoylglyzerin (z.B. CAS Registry Nummer: 620-64-4)

(INCI-Bezeichnung: Triarachidin), enthalten.

Als zweiten wesentlichen Inhaltsstoff enthalten die erfindungsgemäßen Mittel bezogen auf ihr Gewicht 0,05 bis 0,3 Gew.-% Panthenol. Erfindungsgemäß bevorzugte kosmetische oder dermatologische Mittel enthalten 0,06 bis 0,25 Gew.-%, vorzugsweise 0,07 bis 0,2 Gew.-%, weiter bevorzugt 0,08 bis 0,15 Gew.-% und insbesondere 0,09 bis 0,12 Gew.-% Panthenol.

Besonders bevorzugte Mittel enthalten - bezogen auf ihr Gewicht - 0,15 bis 0,45 Gew.-%, vorzugsweise 0,2 bis 0,4 Gew.-%, weiter bevorzugt 0,25 bis 0,35 Gew.-% und insbesondere 0,28 bis 0,32 Gew.-% Linolsäureglycerinester (insbesondere Glyceryltrilinoleat) und 0,06 bis 0,25 Gew.-%, vorzugsweise 0,07 bis 0,2 Gew.-%, weiter bevorzugt 0,08 bis 0,15 Gew.-% und insbesondere 0,09 bis 0,12 Gew.-% Panthenol.

Weitere besonders bevorzugte Mittel enthalten - bezogen auf ihr Gewicht - 0,15 bis 0,45 Gew.-%, vorzugsweise 0,2 bis 0,4 Gew.-%, weiter bevorzugt 0,25 bis 0,35 Gew.-% und insbesondere 0,28 bis 0,32 Gew.-% Linolensäureglycerinester (insbesondere Glyceryltrilinolenat) und 0,06 bis 0,25 Gew.-%, vorzugsweise 0,07 bis 0,2 Gew.-%, weiter bevorzugt 0,08 bis 0,15 Gew.-% und insbesondere 0,09 bis 0,12 Gew.-% Panthenol.

Weitere besonders bevorzugte Mittel enthalten - bezogen auf ihr Gewicht - 0,15 bis 0,45 Gew.-%, vorzugsweise 0,2 bis 0,4 Gew.-%, weiter bevorzugt 0,25 bis 0,35 Gew.-% und insbesondere 0,28 bis 0,32 Gew.-% Arachidonsäureglycerinester (insbesondere Triarachidoylglyzerin) und 0,06 bis 0,25 Gew.-%, vorzugsweise 0,07 bis 0,2 Gew.-%, weiter bevorzugt 0,08 bis 0,15 Gew.-% und insbesondere 0,09 bis 0,12 Gew.-% Panthenol.

Als dritten wesentlichen Inhaltsstoff enthalten die erfindungsgemäßen Mittel bezogen auf ihr Gewicht 0,01 bis 0,1 Tocopherylacetat. Erfindungsgemäß bevorzugte kosmetische oder dermatologische Mittel enthalten 0,02 bis 0,09 Gew.-%, vorzugsweise 0,03 bis 0,08 Gew.-%, weiter bevorzugt 0,04 bis 0,07 Gew.-% und insbesondere 0,05 bis 0,06 Gew.-% Tocopherylacetat.

Besonders bevorzugte Mittel enthalten - bezogen auf ihr Gewicht - 0,15 bis 0,45 Gew.-%, vorzugsweise 0,2 bis 0,4 Gew.-%, weiter bevorzugt 0,25 bis 0,35 Gew.-% und insbesondere 0,28 bis 0,32 Gew.-% Linolsäureglycerinester (insbesondere Glyceryltrilinoleat) und 0,06 bis 0,25 Gew.-%, vorzugsweise 0,07 bis 0,2 Gew.-%, weiter bevorzugt 0,08 bis 0,15 Gew.-% und insbesondere 0,09 bis 0,12 Gew.-% Panthenol und 0,02 bis 0,09 Gew.-%, vorzugsweise 0,03 bis 0,08 Gew.-%, weiter bevorzugt 0,04 bis 0,07 Gew.-% und insbesondere 0,05 bis 0,06 Gew.-% Tocopherylacetat. Weitere besonders bevorzugte Mittel enthalten - bezogen auf ihr Gewicht - 0,15 bis 0,45 Gew.-%, vorzugsweise 0,2 bis 0,4 Gew.-%, weiter bevorzugt 0,25 bis 0,35 Gew.-% und insbesondere 0,28 bis 0,32 Gew.-% Linolensäureglycerinester (insbesondere Glyceryltrilinolenat) und 0,06 bis 0,25 Gew.-%, vorzugsweise 0,07 bis 0,2 Gew.-%, weiter bevorzugt 0,08 bis 0,15 Gew.-% und insbesondere 0,09 bis 0,12 Gew.-% Panthenol und 0,02 bis 0,09 Gew.-%, vorzugsweise 0,03 bis 0,08 Gew.-%, weiter bevorzugt 0,04 bis 0,07 Gew.-% und insbesondere 0,05 bis 0,06 Gew.-% Tocopherylacetat.

Weitere besonders bevorzugte Mittel enthalten - bezogen auf ihr Gewicht - 0,15 bis 0,45 Gew.-%, vorzugsweise 0,2 bis 0,4 Gew.-%, weiter bevorzugt 0,25 bis 0,35 Gew.-% und insbesondere 0,28 bis 0,32 Gew.-% Arachidonsäureglycerinester (insbesondere Triarachidoylglyzerin) und 0,06 bis 0,25 Gew.-%, vorzugsweise 0,07 bis 0,2 Gew.-%, weiter bevorzugt 0,08 bis 0,15 Gew.-% und insbesondere 0,09 bis 0,12 Gew.-% Panthenol und 0,02 bis 0,09 Gew.-%, vorzugsweise 0,03 bis 0,08 Gew.-%, weiter bevorzugt 0,04 bis 0,07 Gew.-% und insbesondere 0,05 bis 0,06 Gew.-% Tocopherylacetat.

Als vierten zwingenden Inhaltsstoff enthalten die erfindungsgemäßen Mittel mindestens ein Harnstoff-Derivat, ausgewählt aus Verbindungen gemäß Formel (I) 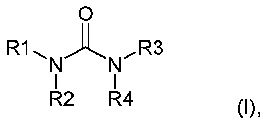 worin
die Reste R1, R2, R3 und R4 unabhängig voneinander für ein Wasserstoffatom, eine C₁-C₄-Alkylgruppe, eine C₂-C₆-Alkenylgruppe oder eine C₂-C₆-Hydroxyalkylgruppe mit mindestens einer Hydroxygruppe steht,
mit der Maßgabe, dass mindestens einer der besagten Reste eine C₂-C₆Hydroxyalkylgruppe mit mindestens einer Hydroxygruppe bedeutet.

Besonders bevorzugte Harnstoffderivate der Formel (I) sind solche, die mindestens eine C₂-C₆-Hydroxyalkylgruppe mit mindestens einer Hydroxygruppe enthalten, welche aus mindestens einem Vertreter aus der Gruppe aus 2-Hydroxyethyl, 2-Hydroxy-2-methylprop-2-yl, 1,3-Dihydroxy-2-methylprop-2-yl, 1,3-Dihydroxyprop-2-yl, Tris(hydroxymethyl)methyl, 1,3-Dihydroxy-2-hydroxymethylprop-2-yl, 2,3-Dihydroxypropyl, 2-Hydroxypropyl, 3-Hydroxypropyl, 4-Hydroxybutyl, 1-Hydroxy-2-methylprop-2-yl, 2,3,4,5,6-Pentahydroxyhexyl und 1,3,4,5,6-Pentahydroxyhex-2-yl ausgewählt wird.

Es ist erfindungsgemäß bevorzugt solche Harnstoffderivate der Formel (I) zu verwenden, welche die bevorzugte Maßgabe der Formel (I) erfüllen, dass mindestens einer der Reste R1 bis R4 eine C₂-C₆-Hydroxyalkylgruppe mit mindestens einer Hydroxygruppe bedeutet. Weiterhin besonders bevorzugt stehen jeweils zwei Reste aus R1, R2, R3 und R4 für eine C₂-C₆-Hydroxyalkylgruppe mit mindestens einer Hydroxygruppe. Insbesondere bevorzugt stehen die zwei Reste aus R1, R2, R3 und R4 für eine C₂-C₆-Hydroxyalkylgruppe mit mindestens einer Hydroxygruppe, wobei diese beiden Reste an unterschiedlichen Stickstoffatomen des Harnstoffs positioniert (N,N'-Stellung) sind. Beispiele für erfindungsgemäß in Verbindungen der Formel (I) verwendbare C₁-C₄-Alkylgruppen sind Methyl, Ethyl, n-Propyl, Isopropyl, n-Butyl, sec-Butyl, Isobutyl und tert-Butyl. Beispiele für erfindungsgemäß in Verbindungen der Formel (I) verwendbare C₂-C₆-Alkenylgruppen sind Vinyl, 2-Propen-1-yl (Allyl) oder 3-Buten-1-yl.

Besonders bevorzugt wird mindestens eine Verbindung aus der Gruppe mit den Vertretern N-(2-Hydroxyethyl)harnstoff, N,N-Bis-(2-hydroxyethyl)harnstoff, N,N'-Bis-(2-hydroxyethyl)harnstoff, N-(3-Hydroxypropyl)harnstoff, N,N-Bis-(3-hydroxypropyl)harnstoff, N,N'-Bis-(3-hydroxypropyl)harnstoff, N-(2-Hydroxypropyl)harnstoff, N,N-Bis-(2-hydroxypropyl)harnstoff, N,N'-Bis-(2-hydroxypropyl)-harnstoff, N-(2-Hydroxy-2-methyl-prop-2-yl)harnstoff, N,N-Bis-(2-hydroxy-2-methyl-prop-2-yl)harnstoff, N,N'-Bis-(2-hydroxy-2-methyl-prop-2-yl)harnstoff, N-(1,3-Dihydroxy-2-methyl-prop-2-yl)harnstoff, N,N-Bis-(1,3-dihydroxy-2-methyl-prop-2-yl)harnstoff, N,N'-Bis-(1,3-dihydroxy-2-methyl-prop-2-yl)harnstoff, N-(1,3-Dihydroxy-2-hydroxymethyl-prop-2-yl)harnstoff, N,N-Bis-(1,3-dihydroxy-2-hydroxymethyl-prop-2-yl)harnstoff und N,N'-Bis-(1,3-dihydroxy-2-hydroxymethyl-prop-2-yl)harnstoff ausgewählt. Ganz besonders bevorzugt ist N-(2-Hydroxyethyl)harnstoff und N,N'-Bis-(2-hydroxyethyl)harnstoff, erhältlich beispielsweise als Handelsprodukt Hydrovance^{®} von der Firma National Starch.

Demnach sind bevorzugte kosmetische oder dermatologische Mittel dadurch gekennzeichnet, dass das Harnstoff-Derivat ausgewählt ist aus N-(2-Hydroxyethyl)harnstoff, N,N-Bis-(2-hydroxyethyl)harnstoff, N,N'-Bis-(2-hydroxyethyl)harnstoff, N-(3-Hydroxypropyl)harnstoff, N,N-Bis-(3-hydroxypropyl)harnstoff, N,N'-Bis-(3-hydroxypropyl)harnstoff, N-(2-Hydroxypropyl)harnstoff, N,N-Bis-(2-hydroxypropyl)harnstoff, N,N'-Bis-(2-hydroxypropyl)harnstoff, N-(2-Hydroxy-2-methyl-prop-2-yl)harnstoff, N,N-Bis-(2-hydroxy-2-methyl-prop-2-yl)harnstoff, N,N'-Bis-(2-hydroxy-2-methyl-prop-2-yl)harnstoff, N-(1,3-Dihydroxy-2-methyl-prop-2-yl)harnstoff, N,N-Bis-(1,3-dihydroxy-2-methyl-prop-2-yl)harnstoff, N,N'-Bis-(1,3-dihydroxy-2-methyl-prop-2-yl)harnstoff, N-(1,3-Dihydroxy-2-hydroxymethyl-prop-2-yl)harnstoff, N,N-Bis-(1,3-dihydroxy-2-hydroxymethyl-prop-2-yl)harnstoff und N,N'-Bis-(1,3-dihydroxy-2-hydroxymethyl-prop-2-yl)harnstoff.

Erfindungsgemäß bevorzugte kosmetische oder dermatologische Mittel sind dadurch gekennzeichnet, dass sie 0,75 bis 4,5 Gew.-%, vorzugsweise 1 bis 4 Gew.-%, weiter bevorzugt 2 bis 3,5 Gew.-% und insbesondere 2,5 bis 3,2 Gew.-% Harnstoff-Derivat(e) der Formel (I) enthalten.

Wie bereits erwähnt, ist N,N'-Bis-(2-hydroxyethyl)harnstoff ein besonders bevorzugter Inhaltsstoff d) für die erfindungsgemäßen Zusammensetzungen. Ganz besonders bevrozugte erfindungsgemäße kosmetische oder dermatologische Mittel sind daher dadurch gekennzeichnet, dass sie 0,75 bis 4,5 Gew.-%, vorzugsweise 1 bis 4 Gew.-%, weiter bevorzugt 2 bis 3,5 Gew.-% und insbesondere 2,5 bis 3,2 Gew.-% N,N'-Bis-(2-hydroxyethyl)harnstoff enthalten.

Besonders bevorzugte Mittel enthalten - bezogen auf ihr Gewicht - 0,15 bis 0,45 Gew.-%, vorzugsweise 0,2 bis 0,4 Gew.-%, weiter bevorzugt 0,25 bis 0,35 Gew.-% und insbesondere 0,28 bis 0,32 Gew.-% Linolsäureglycerinester (insbesondere Glyceryltrilinoleat) und 0,06 bis 0,25 Gew.-%, vorzugsweise 0,07 bis 0,2 Gew.-%, weiter bevorzugt 0,08 bis 0,15 Gew.-% und insbesondere 0,09 bis 0,12 Gew.-% Panthenol und 0,02 bis 0,09 Gew.-%, vorzugsweise 0,03 bis 0,08 Gew.-%, weiter bevorzugt 0,04 bis 0,07 Gew.-% und insbesondere 0,05 bis 0,06 Gew.-% Tocopherylacetat und 0,75 bis 4,5 Gew.-%, vorzugsweise 1 bis 4 Gew.-%, weiter bevorzugt 2 bis 3,5 Gew.-% und insbesondere 2,5 bis 3,2 Gew.-% N,N'-Bis-(2-hydroxyethyl)harnstoff.

Weitere besonders bevorzugte Mittel enthalten - bezogen auf ihr Gewicht - 0,15 bis 0,45 Gew.-%, vorzugsweise 0,2 bis 0,4 Gew.-%, weiter bevorzugt 0,25 bis 0,35 Gew.-% und insbesondere 0,28 bis 0,32 Gew.-% Linolensäureglycerinester (insbesondere Glyceryltrilinolenat) und 0,06 bis 0,25 Gew.-%, vorzugsweise 0,07 bis 0,2 Gew.-%, weiter bevorzugt 0,08 bis 0,15 Gew.-% und insbesondere 0,09 bis 0,12 Gew.-% Panthenol und 0,02 bis 0,09 Gew.-%, vorzugsweise 0,03 bis 0,08 Gew.-%, weiter bevorzugt 0,04 bis 0,07 Gew.-% und insbesondere 0,05 bis 0,06 Gew.-% Tocopherylacetat und 0,75 bis 4,5 Gew.-%, vorzugsweise 1 bis 4 Gew.-%, weiter bevorzugt 2 bis 3,5 Gew.-% und insbesondere 2,5 bis 3,2 Gew.-% N,N'-Bis-(2-hydroxyethyl)harnstoff..

Weitere besonders bevorzugte Mittel enthalten - bezogen auf ihr Gewicht - 0,15 bis 0,45 Gew.-%, vorzugsweise 0,2 bis 0,4 Gew.-%, weiter bevorzugt 0,25 bis 0,35 Gew.-% und insbesondere 0,28 bis 0,32 Gew.-% Arachidonsäureglycerinester (insbesondere Triarachidoylglyzerin) und 0,06 bis 0,25 Gew.-%, vorzugsweise 0,07 bis 0,2 Gew.-%, weiter bevorzugt 0,08 bis 0,15 Gew.-% und insbesondere 0,09 bis 0,12 Gew.-% Panthenol und 0,02 bis 0,09 Gew.-%, vorzugsweise 0,03 bis 0,08 Gew.-%, weiter bevorzugt 0,04 bis 0,07 Gew.-% und insbesondere 0,05 bis 0,06 Gew.-% Tocopherylacetat und 0,75 bis 4,5 Gew.-%, vorzugsweise 1 bis 4 Gew.-%, weiter bevorzugt 2 bis 3,5 Gew.-% und insbesondere 2,5 bis 3,2 Gew.-% N,N'-Bis-(2-hydroxyethyl)harnstoff..

In einer besonders bevorzugten Ausführungsform liegen die erfindungsgemäßen Mittel in Form eines Hydrogels vor. Hydrogele sind Wasser enthaltende Gele auf der Basis hydrophiler, aber wasserunlöslicher Polymere, die als dreidimensionale Netzwerke vorliegen. In Wasser quellen diese Netzwerke unter weitgehender Formerhaltung bis zu einem Gleichgewichtsvolumen. auf. Die Netzwerk-Bildung erfolgt vorwiegend über chemische Verknüpfung der einzelnen Polymerketten, ist aber auch physikalisch durch elektrostatische, hydrophobe oder Dipol/Dipol-Wechselwirkungen zwischen einzelnen Segmenten der Polymerketten möglich. Über die Wahl der zum Polymeraufbau verwendeten Monomeren, die Art der Vernetzung und die Vernetzungsdichte können gewünschte Eigenschaften der Hydrogele gezielt eingestellt werden. Die notwendige Hydrophilie der Polymeren vermitteln unter anderem Hydroxy-, Carboxylat-, Sulfonat- und/oder Amid-Gruppen. Synthetische Hydrogele basieren unter anderem auf Poly(meth)acrylsäuren, Poly(meth)acrylaten, Polyvinylpyrrolidon oder Polyvinylalkohol. Bevorzugte anionische polymere Hydrogelbildner, die die Wirkung des erfindungsgemäß verwendeten Wirkstoffs unterstützen können, enthalten Carboxylat- und/oder Sulfonatgruppen und als Monomere zum Beispiel Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind 2-Acryl-amido-2-methylpropansulfonsäure und Acrylsäure. Ganz besonders bevorzugte anionische Polymere enthalten als alleiniges Monomer oder als Comonomer 2-Acrylamido-2-methylpropansulfonsäure, wobei die Sulfonsäuregruppe ganz oder teilweise in Salzform vorliegen kann. Innerhalb dieser Ausführungsform ist es bevorzugt, Copolymere aus mindestens einem anionischen Monomer und mindestens einem nichtionischen Monomer einzusetzen. Bezüglich der anionischen Monomere wird auf die oben aufgeführten Substanzen verwiesen. Bevorzugte nichtionische Monomere sind Acrylamid, Methacrylamid, Acrylsäureester, Methacrylsäureester, Vinylpyrrolidon, Vinylether und Vinylester. Bevorzugte anionische Copolymere sind Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppen ganz oder teilweise als Natrium-, Kalium-, Ammonium-, Mono- oder Triethanolammonium-Salz vorliegen. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylen-bisacrylamid zum Einsatz kommen. Ein solches Polymer ist in dem Handelsprodukt Sepigel^{®}305 der Firma SEPPIC enthalten. Die Verwendung dieses Compounds hat sich im Rahmen der erfindungsgemäßen Lehre als besonders vorteilhaft erwiesen. Auch die unter der Bezeichnungen Simulgel^{®} 600, Simulgel^{®} NS, Simulgel^{®} EG, Simulgel^{®} EPG und Sepiplus^{®} 400 als Compound mit einem Öl wie Isohexadecan, Squalan oder hydriertem Polyisobuten, und einem Emulgator, wie Polysorbate, Laureth-7 oder Caprylyl/Capryl Glucoside, vertriebenen Natriumacryloyldimethyltaurat-Copolymere (mit Monomeren, ausgewählt aus Acrylamid, Natriumacrylat, Hydroxyethylacrylat und/oder Acrylsäure) haben sich als erfindungsgemäß besonders wirksam erwiesen. Weitere bevorzugte Hydrogelbildner sind Ammoniumacryloyldimethyltaurat-Vinylpyrrolidon-Copolymere, insbesondere das Handelsprodukt Aristoflex^{®} AVC von Clariant.

Weitere besonders bevorzugte anionische Homopolymer-Hydrogelbildner sind unvernetzte und vernetzte Polyacrylsäuren. Dabei können Allylether von Pentaerythrit, von Sucrose und von Propylen bevorzugte Vernetzungsagentien sein. Solche Verbindungen sind zum Beispiel die Handelsprodukte Carbopol^{®}. Ein besonders bevorzugtes anionisches Copolymer enthält als Monomer zu 80 - 98 % eine ungesättigte C₃₋₆-Carbonsäure oder ihr Anhydrid sowie zu 2 - 20 % Acrylsäureester von gesättigten C₁₀₋₃₀-Carbonsäuren, wobei das Copolymer mit den vorgenannten Vernetzungsagentien vernetzt sein kann. Entsprechende Handelsprodukte sind Pemulen^{®} und die Carbopol^{®}-Typen 954, 980, 1342 und ETD 2020 (ex Noveon bzw. B.F. Goodrich).

Bevorzugte nichtionische polymere Hydrogelbildner sind beispielsweise Polyvinylalkohole, die teilverseift sein können, z. B. die Handelsprodukte Mowiol^{®} sowie Vinylpyrrolidon/Vinylester-Copolymere und Polyvinylpyrrolidone, die z. B. unter dem Warenzeichen Luviskol^{®} (BASF) vertrieben werden.

Erfindungsgemäß bevorzugte natürliche Hydrogelbildner sind insbesondere ausgewählt aus gewünschtenfalls physikalisch (thermisch) und/oder chemisch modifizierten Cellulose-Derivaten, vorzugsweise Hydroxyalkylcellulosen wie Hydroxyethylcellulose, Hydroxypropylcellulose, Hydroxypropylmethylcellulose, Methylcellulose, Hydroxymethylcellulose, Carboxymethylcellulose weiterhin insbesondere Stärken sowie Stärkeabbauprodukte wie Amylose und Amylopektin, chemisch und/oder thermisch modifiziertenStärken, z. B. Hydroxypropylstärkephosphat, Dihydroxypropyldistärkephosphat, insbesondere den anionischen Stärkederivaten Aluminiumstärkeoctenylsuccinat (z.B. die Handelsprodukte Dry Flo^{®}), Natriumstärkeoctenylsuccinat, Calciumstärkeoctenylsuccinat, weiterhin Chitosan und dessen Derivaten, weiterhin Polysacchariden, die Gums oder Gummen bilden, wie beispielsweise Guar-Gum, Xanthan-Gum, Alginate, insbesondere Natriumalginat, Gummi arabicum, Karaya-Gummi, Carrageenane, Johannisbrotkernmehl, Leinsamen-Gums, Schellack und Agar-Agar.

In einer weiteren besonders bevorzugten Ausführungsform liegen die erfindungsgemäßen Mittel in Form einer Öl-in-Wasser-Emulsion vor. Derartige Öl-in-Wasser-Emulsionen können, ebenfalls bevorzugt, durch mindestens einen der vorstehend genannten Hydrogelbildner stabilisiert vorliegen.

In einer weiteren besonders bevorzugten Ausführungsform liegen die erfindungsgemäßen Mittel in flüssiger Form vor. Die Applikation kann dabei vorzugsweise mit Sprühvorrichtungen erfolgen. Diese Sprühvorrichtungen enthalten in einem Behälter eine Füllung aus dem erfindungsgemäßen (flüssigen, breiartigen oder pulverförmigen) Hautbehandlungsmittel. Die Füllung kann unter dem Druck eines Treibmittels stehen (Druckgasdosen, Druckgaspackungen, Aerosolpackungen), oder es kann sich um einen mechanisch zu bedienenden Pumpzerstäuber (Pumpsprays) handeln. Die Behälter weisen eine Entnahmevorrichtung auf, vorzugsweise in Gestalt von Ventilen, die die Entnahme des Inhalts als Nebel, Rauch, Schaum, Pulver, Paste oder Flüssigkeitsstrahl ermöglichen. Als Behälter für die Sprühvorrichtungen kommen vor allem zylindrische Gefäße aus Metall (Aluminium, Weißblech, Rauminhalt vorzugsweise <1000 ml), geschütztem bzw. nichtsplitterndem Glas oder Kunststoff (Rauminhalt vorzugsweise <220 ml) bzw. splitterndem Glas oder Kunststoff (Rauminhalt vorzugsweise <150 ml) in Frage.

Cremeförmige, gelförmige, pastose und flüssige Mittel können z.B. in Pump- oder Quetschspendern abgepackt sein, insbesondere auch in Mehrkammer-Pump- oder Quetschspendern.

Unter den Begriff der Flüssigkeit fallen im Sinne der Erfindung auch jegliche Festkörperdispersionen in Flüssigkeiten. Erfindungsgemäße Mittel können auch als Pasten, Salben, Lotionen oder Cremes vorliegen. Feste Mittel können beispielsweise als loser Puder, gepresster Puder oder als Stift vorliegen.

Die Applikation kann z.B. im Falle flüssiger Mittel vorzugsweise auch mit einem Roller-Applikator erfolgen, wie er z.B. aus dem Bereich der Deo-Roller bekannt ist. Solche Roller weisen eine in einem Kugelbett gelagerte Kugel auf, welche durch Bewegung über eine Oberfläche bewegt werden kann. Dabei nimmt die Kugel etwas von dem zu verteilenden Mittel auf und befördert dieses an die zu behandelnde Oberfläche. Mit derartigen Applikatoren lassen sich gezielt und sparsam nur die tatsächlich von Akne und/oder Unreinheiten betroffenen Hautpartien behandeln.

Die Applikation kann z.B. auch mit Substraten erfolgen, die mit einer erfindungsgemäßen Zubereitung beaufschlagt sind. Besonders bevorzugt sind Feuchttücher, d.h. für den Anwender vorgefertigte, vorzugsweise einzeln abgepackte, Feuchttücher, wie sie z. B. aus dem Bereich der Glasreinigung (Brillenputztücher) oder aus dem Bereich der feuchten Toilettenpapiere wohlbekannt sind. Solche Feuchttücher, die vorteilhafterweise auch Konservierungsstoffe enthalten können, sind dann vorteilhafterweise mit einem erfindungsgemäßen Mittel imprägniert oder beaufschlagt, vorteilhafterweise sind sie einzeln verpackt. Sie können z. B. als Reinigungs-Tuch oder Abschminktuch (Abschminkpad) eingesetzt werden, was besonders interessant für den Gebrauch unterwegs ist.

Bevorzugte Substratmaterialien sind bevorzugt ausgewählt aus porösen flächigen Tüchern. Sie können aus einem faserigen oder zellulären flexiblen Material bestehen, das ausreichend mechanische Stabilität und gleichzeitig Weichheit zur Anwendung auf der Haut aufweist. Zu diesen Tüchern gehören Tücher aus gewebtem und ungewebtem synthetischen und natürlichen Fasern, Filz, Papier oder Schaumstoff, wie hydrophilem Polyurethanschaum.

Vorzugsweise werden hier herkömmliche Tücher aus ungewebtem Material (Vliese) verwendet. Vliese sind im allgemeinen als adhäsiv gebondete faserige Produkte definiert, die eine Matte oder geschichtete Faserstruktur aufweisen, oder solche, die Fasermatten umfassen, bei denen die Fasern zufällig oder in statistischer Anordnung verteilt sind. Die Fasern können natürlich sein, wie Wolle, Seide, Jute, Hanf, Baumwolle, Lein, Sisal oder Ramie; oder synthetisch, wie Rayon, Celluloseester, Polyvinylderivate, Polyolefine, Polyamide oder Polyester. Im Allgemeinen ist jeder Faserdurchmesser bzw. -titer für die vorliegende Erfindung geeignet. Die hier eingesetzten ungewebten Stoffe neigen aufgrund der zufälligen oder statistischen Anordnung von Fasern in dem ungewebten Material, die ausgezeichnete Festigkeit in allen Richtungen verleihen, nicht zum Zerreißen oder Zerfallen. Beispiele für ungewebte Stoffe, die sich als Substrate in der vorliegenden Erfindung eignen, sind beispielsweise aus WO 98/18441 bekannt. Bevorzugte poröse und flächige Reinigungstücher bestehen aus einem oder verschiedenen Fasermaterialien, insbesondere aus Baumwolle, veredelter Baumwolle, Polyamid, Polyester oder Mischungen aus diesen. Vorzugsweise weisen die Substrate in Tuchform eine Fläche von 10 bis 5000 cm², vorzugsweise von 50 bis 2000 cm², insbesondere von 100 bis 1500 cm² und besonders bevorzugt von 200 bis 1000 cm² auf. Die Grammatur des Materials beträgt dabei üblicherweise zwischen 20 und 1000 g/m², vorzugsweise von 30 bis 500 g/m² und insbesondere von 50 bis 150 g/m². Erfindungsgemäß bevorzugte Hautbehandlungs-Substrate können durch Tränken oder Imprägnierung oder auch durch Aufschmelzen der erfindungsgemäßen Mittel auf ein Substrat erhalten werden.

Die erfindungsgemäßen Mittel, vorzugsweise flüssigen Mittel, können auch mehrphasig sein, die Phasen können z.B. horizontal, also übereinander, oder vertikal, also nebeneinander, angeordnet sein. Es kann sich auch um ein disperses System handeln, bei dem z.B. die festen Bestandteile inhomogen in der flüssigen Matrix verteilt sind, so dass ein solches disperses System vor der Anwendung geschüttelt werden sollte.

Je nach Darreichungsform enthalten die erfindungsgemäßen Zusammensetzungen weitere Wirk-und/oder Hilfsstoffe.

Wie bereits erwähnt, enthalten die erfindungsgemäßen Zusammensetzungen vorzugsweise weitere Inhaltstoffe. Im Hinblick auf die Behandlung trockener, rissiger Haut haben sich bestimmte weitere Wirksubstanzen als besonders bevorzugt erwiesen, da sie hautpflegende und hautberuhigende Eigenschaften haben und in ihrer Wirkung durch die erfindungsgemäß eingesetzten Hauptwirksubstanzen verstärkt werden.

Erfindungsgemäß besonders bevorzugte Zusammensetzungen enthalten weiterhin mindestens einen Wirkstoff, ausgewählt aus:
a) Monomeren, Oligomeren und Polymeren von Aminosäuren, N-C₂-C₂₄-Acylaminosäuren, den Estern und/oder den physiologisch verträglichen Metallsalzen dieser Substanzen,
b) DNA- oder RNA-Oligonucleotiden,
c) natürlichen Betainverbindungen,
d) Vitaminen, Provitaminen und Vitaminvorstufen der Gruppen A, B, C, E, H und K und den Estern der vorgenannten Substanzen,
e) α-Hydroxycarbonsäuren, α-Ketocarbonsäuren, β-Hydroxycarbonsäuren und deren Ester-, Lacton- oder Salzform, ausgenommen Zusammensetzungen mit 5 Gew.-% (2-Hydroxyethyl)harnstoff und 0,05 bzw. 5 Gew.-% Ammoniumlactat und Zusammensetzungen mit (2-Hydroxypropyl)harnstoff und α- und/oder β-Hydroxycarbonsäuren,
f) den Flavonoiden Naringin, α-Glucosylrutin, α-Glucosylmyricetin, α-Glucosylisoquercetin, α-Glucosylquercetin, Dihydroquercetin (Taxifolin), Hesperidin, Hesperitin, Neohesperidin, Rutin, Troxerutin, Monoxerutin, Diosmin, Eriodictin und Apigenin-7-glucosid,
g) Isoflavonoiden und Isoflavonoid-reichen Pflanzenextrakten,
h) Ubichinon und Ubichinol sowie deren Derivaten,
i) Silymarin,
j) natürlich vorkommenden Xanthin-Derivaten, ausgewählt aus Coffein, Theophyllin, Theobromin und Aminophyllin, besonders bevorzugt Koffein,
k) Ectoin,
l) Kreatin,
m) Olivenblattextrakten, Ursolsäure, Oleanol und/oder Oleanolsäure,
n) Mono- und Polyhydroxystilbenen und deren Estern,
o) Derivaten von methyliertem Silanol,
p) Phytinsäure,
q) Extrakten aus Maiskörnern (Zea Mays (Corn) Kernel Extract),
r) Extrakten aus Haferkörnern (Avena Sativa (Oat) Kernel Extract),
s) Saccharomyces/Xylinum/Black Tea Ferment,
t) Apfelkernextrakten (Pyrus Malus (Apple) Fruit Extract),
u) Lotuskeim-Extrakten (Nelumbo Nucifera Germ Extract),
v) Rotweinextrakten,
w) Traubenkernextrakten (Vitis Vinifera (Grape) Seed Extract), die bevorzugt aus der Chardonnay-Traube stammen,
x) Extrakten aus Schwarzen Holunderblüten (Sambucus Nigra Flower Extract),
y) Wirkstoffen, die die beta-Endorphinsynthese in Keratinozyten stimulieren,
z) anorganischen und organischen UV-Filtersubstanzen,
aa) selbstbräunenden Wirkstoffen,
bb) hautaufhellenden Wirkstoffen,
cc) Wirkstoffen, die die Prostaglandinsynthese und/oder die Leukotrien-Synthese inhibieren,
dd) sebumregulierenden Wirkstoffen,
ee) sowie Desoxyzuckern oder Desoxyzucker-Bausteine enthaltenden Polysacchariden.

In einer ersten bevorzugten Ausführungsform enthalten die erfindungsgemäßen Zusammensetzungen neben den zwingenden Inhaltsstoffen a) bis d) mindestens ein Monomer, Oligomer oder Polymer von Aminosäuren, N-C₂-C₂₄-Acylaminosäuren und/oder den Estern und/oder den physiologisch verträglichen Metallsalzen dieser Substanzen.

Die Monomere der Aminosäuren und/oder der N-C₂-C₂₄-Acylaminosäuren sind ausgewählt aus Alanin, Arginin, Asparagin, Asparaginsäure, Canavanin, Citrullin, Cystein, Cystin, Desmosin, Glutamin, Glutaminsäure, Glycin, Histidin, Hydroxylysin, Hydroxyprolin, Isodesmosin, Isoleucin, Leucin, Lysin, Methionin, Ornithin, Phenylalanin, Prolin, Pyroglutaminsäure, Sarcosin, Serin, Taurin, Threonin, Thyroxin, Tryptophan, Tyrosin und Valin. Besonders bevorzugt sind Taurin, Arginin, Hydroxyprolin, Prolin und Glutaminsäure.

Der C₂ - C₂₄-Acylrest, mit dem die genannten Aminosäuren an der Aminogruppe derivatisiert sind, ist ausgewählt aus einem Acetyl-, Propanoyl-, Butanoyl-, Pentanoyl-, Hexanoyl-, Heptanoyl-, Octanoyl-, Nonanoyl-, Decanoyl-, Undecanoyl-, Lauroyl-, Tridecanoyl-, Myristoyl-, Pentadecanoyl-, Cetoyl-, Palmitoyl-, Stearoyl-, Elaidoyl-, Arachidoyl- oder Behenoyl-Rest. Mischungen von C₈-C₁₈-Acylresten werden auch als Cocoyl-Rest bezeichnet und sind ebenfalls bevorzugte Substituenten. Bevorzugte Beispiele sind Zinkpyroglutamat, Natriumoctanoylglutamat, Natriumdecanoylglutamat, Natriumlauroylglutamat, Natriummyristoylglutamat, Natriumcetoylglutamat und Natriumstearoylglutamat. Besonders bevorzugt sind Zinkpyroglutamat und Natriumlauroylglutamat.

Die physiologisch verträglichen Salze der erfindungsgemäß bevorzugten Wirkstoffe, die Säuregruppen enthalten und Salze bilden können, sind ausgewählt aus den Ammonium-, Alkalimetall-, Magnesium-, Calcium-, Aluminium-, Zink- und Mangan-Salzen. Bevorzugt sind die Natrium-, Kalium-, Magnesium-, Aluminium-, Zink- und Mangan-Salze.

Die Oligomere der Aminosäuren und/oder der N-C₂-C₂₄-Acylaminosäuren sind ausgewählt aus Di-, Tri-, Tetra-, Penta-, Hexa-, Hepta-, Octa-, Nona- und Decapeptiden, die acyliert und/oder verestert sein können. Besonders bevorzugt sind die Di-, Tri-, Tetra-, Penta- und Hexapeptide, die acyliert und/oder verestert sein können. Erfindungsgemäß bevorzugte, gegebenfalls acylierte und/oder veresterte Dipeptide sind Tyr-Arg, Val-Trp, Asn-Phe, Asp-Phe, N-Palmitoyl-ß-Ala-His, N-Acetyl-Tyr-Arg-hexyldecylester (z.B. Calmosensine von Sederma), Carnosin (ß-Ala-His) und N-Palmitoyl-Pro-Arg. Erfindungsgemäß bevorzugte, gegebenfalls acylierte und/oder veresterte Tripeptide sind Gly-His-Lys, N-Palmitoyl-Gly-His-Lys, Gly-Lys-His, His-Ala-Orn, Lys-Phe-Lys, N-Elaidoyl-Lys-Phe-Lys und N-Acetyl-Arg-Lys-Arg-NH₂. Erfindungsgemäß bevorzugte, gegebenfalls acylierte und/oder veresterte Tetrapeptide sind Gly-Gln-Pro-Arg, Gly-Gln-Arg-Pro und N-Palmitoyl-Gly-Gln-Pro-Arg. Erfindungsgemäß bevorzugte, gegebenfalls acylierte und/oder veresterte Pentapeptide sind Lys-Thr-Thr-Lys-Ser, N-Palmitoyl-Lys-Thr-Thr-Lys-Ser, N-Palmitoyl-Tyr-Gly-Gly-Phe-Met und N-Palmitoyl-Tyr-Gly-Gly-Phe-Leu. Ein erfindungsgemäß bevorzugtes Hexapeptid ist Palmitoyl-Val-Gly-Val-Ala-Pro-Gly (Biopeptide EL von Sederma).

Es kann erfindungsgemäß besonders bevorzugt sein, ein Gemisch aus mindestens zwei Oligopeptiden einzusetzen. Ein besonders bevorzugtes Gemisch ist die Kombination aus N-Palmitoyl-Gly-His-Lys (z.B. Biopeptide CL von Sederma) und N-Palmitoyl-Gly-Gln-Pro-Arg (z.B. in Eyeliss von Sederma). Eine vorgefertigte Mischung des Tripeptids Palmitoyl-Gly-His-Lys und des Tetrapeptids N-Palmitoyl-Gly-Gln-Pro-Arg ist unter dem Handelsnamen Matrixyl 3000, ebenfalls von Sederma, erhältlich.

Die Polymere der Aminosäuren und/oder der N-C₂-C₂₄-Acylaminosäuren sind ausgewählt aus pflanzlichen und tierischen Proteinhydrolysaten und/oder Proteinen. Tierische Proteinhydrolysate sind z. B. Elastin-, Collagen-, Keratin-, Seiden- und Milcheiweiß-Proteinhydrolysate, die auch in Form von Salzen vorliegen können. Erfindungsgemäß bevorzugt sind pflanzliche Proteinhydrolysate, z. B. Soja-, Weizen-, Mandel-, Erbsen-, Kartoffel- und Reisproteinhydrolysate. Entsprechende Handelsprodukte sind z. B. DiaMin^{®} (Diamalt), Gluadin^{®} (Cognis), Lexein^{®} (Inolex) und Crotein^{®} (Croda). Besonders bevorzugt sind Sojaproteinhydrolysate, z. B. die Handelsprodukte Phytokine^{®} von Coletica oder Ridulisse C^{®} von Silab.

Proteinhydrolysate können naturgemäß auch monomere Aminosäuren und Oligopeptide enthalten; ihre Zusammensetzung ist normalerweise nicht definiert.

Ebenfalls möglich ist der Einsatz von Acylderivaten der Proteinhydrolysate, z. B. in Form ihrer Fettsäure-Kondensationsprodukte. Entsprechende Handelsprodukte sind z. B. Lamepon^{®} (Cognis), Gluadin^{®} (Cognis), Lexein^{®} (Inolex), Crolastin^{®} oder Crotein^{®} (Croda).

Erfindungsgemäß einsetzbar sind auch kationisierte Proteinhydrolysate. Bevorzugt sind kationische Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Weiterhin können die kationischen Proteinhydrolysate auch noch weiter derivatisiert sein. Als typische Beispiele für erfindungsgemäß verwendete kationische Proteinhydrolysate und -derivate sind einige der unter den INCI- Bezeichnungen im "International Cosmetic Ingredient Dictionary and Handbook", (seventh edition 1997, The Cosmetic, Toiletry, and Fragrance Association 1101 17th Street, N.W., Suite 300, Washington, DC 20036-4702) genannten und im Handel erhältlichen Produkte aufgeführt: Cocodimonium Hydroxypropyl Hydrolyzed Collagen, Steardimonium Hydroxypropyl Hydrolyzed Collagen, Cocodimonium Hydroxypropyl Hydrolyzed Rice Protein, Cocodimonium Hydroxypropyl Hydrolyzed Silk, Cocodimonium Hydroxypropyl Hydrolyzed Soy Protein, Cocodimonium Hydroxypropyl Hydrolyzed Wheat Protein, Cocodimonium Hydroxypropyl Silk Amino Acids, Hydroxypropyl Arginine Lauryl/Myristyl Ether HCl. Ganz besonders bevorzugt sind die kationischen Proteinhydrolysate und -derivate auf pflanzlicher Basis.

In einer weiteren bevorzugten Ausführungsform sind die Polymeren der Aminosäuren ausgewählt aus DNA-Reparaturenzymen.

Erfindungsgemäß bevorzugte DNA-Reparaturenzyme sind Photolyase und T4 Endonuclease V, letztere im Weiteren mit "T4N5" abgekürzt. Diese beiden Enzyme sind im Stand der Technik bereits als so genannte DNA-Reparatur-Enzyme bekannt. Unter DNA-Reparatur ist definitionsgemäß die Spaltung bzw. Entfernung von UV-induzierten Pyrimidindimeren aus der DNA zu verstehen.

Photolyase ist die Kurzbezeichnung für Desoxyribodipyrimidin-Photolyase bzw. DNA-Photolyase, ein Enzym mit der Klassifizierungsnummer EC 4.1.99.3. Eine besonders effiziente Photolyase stammt aus *Anacystis nidulans,* einem phototrophen marinen Mikroorganismus. Die Photolyase aus *A. nidulans* wird in technisch relevanten Mengen mittlerweile aus E. coli gewonnen. Photolyase ist zur Aktivierung auf Licht angewiesen. Das Enzym T4 Endonuclease V wird vom denV-Gen der Bakteriophage T4 produziert und gehört zu den Phosphodiesterasen, die die Nucleinsäuren an der (5'-3')-Bindung hydrolytisch spalten. T4N5 ist auch ohne Lichteinfluss aktiv.

Erfindungsgemäß besonders bevorzugt ist der Einsatz von liposomenverkapselten DNA-Reparaturenzymen. Liposomenverkapselte Photolyase ist im Handel z. B. unter der Produktbezeichnung Photosome™, liposomenverkapselte T4N5 z. B. unter der Bezeichnung Ultrasome™ von der Firma AGI Dermatics, USA, erhältlich.

In den erfindungsgemäßen Zusammensetzungen sind die Photosome™ oder Ultrasome™ in Mengen von 0,1 - 10 Gew.-%, bevorzugt 0,5 - 5,0 Gew.-% und besonders bevorzugt 1,0 - 4,0 Gew.-%, bezogen auf das gesamte Mittel, enthalten.

In den erfindungsgemäßen Zusammensetzungen sind die Monomere, Oligomere oder Polymere von Aminosäuren, N-C₂-C₂₄-Acylaminosäuren und/oder den Estern und/oder den physiologisch verträglichen Metallsalzen dieser Substanzen in Mengen von 0,0001 - 10 Gew.-%, bevorzugt 0,01 - 5 Gew.-% und besonders bevorzugt 0,1 - 3 Gew.-%, jeweils bezogen auf die gesamte Zusammensetzung, enthalten.

In einer weiteren bevorzugten Ausführungsform enthalten die erfindungsgemäßen Zusammensetzungen neben den zwingenden Inhaltsstoffen a) bis d) mindestens ein DNA-Oligonucleotid oder mindestens ein RNA-Oligonucleotid.

Erfindungsgemäß werden unter einem Oligonucleotid Polymerisate aus 2 bis 20, bevorzugt 2 bis 10 Mononucleotiden verstanden, die ebenso wie bei Polynucleotiden und Nucleinsäuren durch Phosphorsäurediester-Brücken verknüpft sind. Die Nucleotide bestehen aus Nucleobasen (meist Pyrimidin- oder Purin-Derivaten), Pentosen (meist D-Ribofuranose oder 2-Desoxy-D-ribofuranose in β-N-glykosidischer Bindung an die Nucleobase) und Phosphorsäure. Die Mononucleotide sind zum Beispiel Adenosinphosphate, Cytidinphosphate, Guanosinphosphate, Uridinphosphate und Thymidinphosphate, insbesondere CMP (Cytidin-5'-monophosphat), UDP (Uridin-5'-diphosphat), ATP (Adenosin-5 '-triphosphat) und GTP (Guanosin-5'-triphosphat).

Ein erfindungsgemäß besonders bevorzugtes Oligonucleotid ist das Thymidin-Dinucleotid.

In den erfindungsgemäßen Zusammensetzungen sind die DNA-Oligonucleotide oder RNA-Oligonucleotide vorzugsweise in Mengen von 0,0001 - 5 Gew.-%, bevorzugt 0,001 - 1,0 Gew.-% und besonders bevorzugt 0,01 - 0,5 Gew.-%, bezogen auf das gesamte Mittel, enthalten.

In einer weiteren bevorzugten Ausführungsform enthalten die erfindungsgemäßen Zusammensetzungen neben den zwingenden Inhaltsstoffen a) bis d) mindestens eine natürliche Betainverbindung. Erfindungsgemäße natürliche Betainverbindungen sind natürlich vorkommende Verbindungen mit der Atomgruppierung R₃N⁺-CH₂-X-COO⁻ gemäß IUPAC-Regel C-816.1. So genannte Betaintenside (synthetisch) fallen nicht unter die erfindungsgemäß verwendeten Betainverbindungen, ebenso wenig andere zwitterionische Verbindungen, in denen sich die positive Ladung an N oder P und die negative Ladung formal an O, S, B oder C befindet, die aber nicht der IUPAC-Regel C-816.1 entsprechen. Erfindungsgemäß bevorzugte Betainverbindungen sind Betain (Me₃N⁺-CH₂-COO⁻) und Carnitin (Me₃N⁺-CH₂-CHOH-CH₂-COO⁻), jeweils mit Me = Methyl und X = C-C-Einfachbindung (im Falle des Betains) oder X = -CHOH-CH₂- (im Falle des Carnitins).

Die Betainverbindungen sind in den erfindungsgemäßen Mitteln vorzugsweise in einer Gesamtmenge von 0,05 bis 5 Gew.-%, bevorzugt 0,1 bis 3 Gew.-%, besonders bevorzugt 0,5 bis 2 Gew.-%, jeweils bezogen auf die gesamte Zusammensetzung, enthalten.

In einer weiteren bevorzugten Ausführungsform enthalten die erfindungsgemäßen Zusammensetzungen neben den zwingenden Inhaltsstoffen a) bis d) mindestens ein Vitamin, Provitamin oder eine als Vitaminvorstufe bezeichnete Verbindung aus den Vitamingruppen A, B, C, E, H und K und den Estern der vorgenannten Substanzen.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester, wie Retinylpalmitat und Retinylacetat in Betracht. Die erfindungsgemäßen Zubereitungen enthalten die Vitamin A-Komponente bevorzugt in Mengen von 0,05 - 1 Gew.-%, bezogen auf die gesamte Zusammensetzung.

Zur Vitamin B-Gruppe oder zu dem Vitamin B-Komplex gehören unter anderem
- Vitamin B₁, Trivialname Thiamin, chemische Bezeichnung 3-[(4'-Amino-2'-methyl-5'-pyrimidinyl)-methyl]-5-(2-hydroxyethyl)-4-methylthiazoliumchlorid. Bevorzugt wird Thiaminhydrochlorid in Mengen von 0,05 bis 1 Gew.-%, bezogen auf das gesamte Mittel, eingesetzt.
- Vitamin B₂, Trivialname Riboflavin, chemische Bezeichnung 7,8-Dimethyl-10-(1-D-ribityl)-benzo[g]pteridin-2,4(3*H*,10*H*)-dion. Bevorzugt werden Riboflavin oder seine Derivate in Mengen von 0,05 bis 1 Gew.-%, bezogen auf das gesamte Mittel, eingesetzt.
- Vitamin B₃. Unter dieser Bezeichnung werden die Verbindungen Nicotinsäure und Nicotinsäureamid (Niacinamid) geführt. Erfindungsgemäß bevorzugt ist das Nicotinsäureamid, das in den erfindungsgemäßen Mitteln bevorzugt in Mengen von 0,05 bis 1 Gew.-%, bezogen auf das gesamte Mittel, enthalten ist.
- Vitamin B₅ (Pantothensäure und Panthenol). Panthenol ist zwingender Inhaltsstoff b) in den erfindungsgemäßen Mitteln. Eventuell in den Mittel enthaltene Panthothensäure oder Panthenolderivate werden nicht in die Mengen an Inhaltsstoff b) eingerechnet. Erfindungsgemäß einsetzbare Derivate des Panthenols sind insbesondere die Ester und Ether des Panthenols sowie kationisch derivatisierte Panthenole. In einer weiteren bevorzugten Ausführungsform der Erfindung können an Stelle von sowie zusätzlich zu Pantothensäure oder Panthenol auch Derivate des 2-Furanon mit der allgemeinen Strukturformel (II) eingesetzt werden.

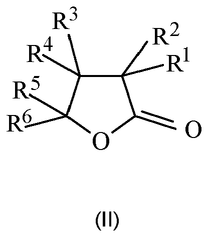

Bevorzugt sind die 2-Furanon-Derivate, in denen die Substituenten R¹ bis R⁶ unabhängig voneinander ein Wasserstoffatom, einen Hydroxylrest, einen Methyl-, Methoxy-, Aminomethyl-oder Hydroxymethylrest, einen gesättigten oder ein- oder zweifach ungesättigten, linearen oder verzweigten C₂-C₄ - Kohlenwasserstoffrest, einen gesättigten oder ein- oder zweifach ungesättigten, verzweigten oder linearen Mono-, Di- oder Trihydroxy-C₂-C₄ - Kohlenwasserstoffrest oder einen gesättigten oder ein- oder zweifach ungesättigten, verzweigten oder linearen Mono-, Di- oder Triamino-C₂-C₄ - Kohlenwasserstoffrest darstellen. Besonders bevorzugte Derivate sind die auch im Handel erhältlichen Substanzen Dihydro-3-hydroxy-4,4-dimethyl-2(3H)-furanon mit dem Trivialnamen Pantolacton (Merck), 4-Hydroxymethyl-γ-butyrolacton (Merck), 3,3-Dimethyl-2-hydroxy-γ-butyrolacton (Aldrich) und 2,5-Dihydro-5-methoxy-2-furanon (Merck), wobei ausdrücklich alle Stereoisomeren eingeschlossen sind. Das erfindungsgemäß außerordentlich bevorzugte 2-Furanon-Derivat ist Pantolacton (Dihydro-3-hydroxy-4,4-dimethyl-2(3H)-furanon), wobei in Formel (I) R¹ für eine Hydroxylgruppe, R² für ein Wasserstoffatom, R³ und R⁴ für eine Methylgruppe und R⁵ und R⁶ für ein Wasserstoffatom stehen. Das Stereoisomer (R)-Pantolacton entsteht beim Abbau von Pantothensäure.

Die genannten Verbindungen des Vitamin B₅-Typs sowie die 2-Furanonderivate sind in den erfindungsgemäßen Mitteln in einer Gesamtmenge von 0,05 bis 5 Gew.-%, bevorzugt 0,1 bis 3 Gew.-%, besonders bevorzugt 0,5 bis 2 Gew.-%, jeweils bezogen auf die gesamte Zusammensetzung, enthalten.
- Vitamin B₆, wobei man hierunter keine einheitliche Substanz, sondern die unter den Trivialnamen Pyridoxin, Pyridoxamin und Pyridoxal bekannten Derivate des 5-Hydroxymethyl-2-methylpyridin-3-ols versteht. Vitamin B₆ ist in den erfindungsgemäßen Mitteln bevorzugt in Mengen von 0,0001 bis 1,0 Gew.-%, insbesondere in Mengen von 0,001 bis 0,01 Gew.-%, enthalten.
- Vitamin B₇ (Biotin), auch als Vitamin H oder "Hautvitamin" bezeichnet. Bei Biotin handelt es sich um (3a*S*,4*S*, 6a*R*)-2-Oxohexahydrothienol[3,4-d]-imidazol-4-valeriansäure. Biotin ist in den erfindungsgemäßen Mitteln bevorzugt in Mengen von 0,0001 bis 1,0 Gew.-%, insbesondere in Mengen von 0,001 bis 0,01 Gew.-% enthalten.

Vitamin C (Ascorbinsäure) wird bevorzugt in Mengen von 0,1 bis 3 Gew.-%, bezogen auf die gesamte Zusammensetzung, eingesetzt. Die Verwendung der Derivate Ascorbylpalmitat, -stearat, - dipalmitat, -acetat, Mg-Ascorbylphosphat, Na-Ascorbylphosphat, Natrium- und Magnesiumascorbat, Dinatriumascorbylphosphat und -sulfat, Kaliumascorbyltocopherylphosphat, Chitosanascorbat oder Ascorbylglucosid kann bevorzugt sein. Die Verwendung in Kombination mit Tocopherolen kann ebenfalls bevorzugt sein.

Zur Vitamin E-Gruppe zählen Tocopherol, insbesondere α-Tocopherol, und seine Derivate. Bevorzugte Derivate sind insbesondere die Ester, wie Tocopherylacetat, -nicotinat, -phosphat, - succinat, -linoleat, -oleat, Tocophereth-5, Tocophereth-10, Tocophereth-12, Tocophereth-18, Tocophereth-50 und Tocophersolan. Tocopherol und seine Derivate sind bevorzugt in Mengen von 0,05 - 1 Gew.-%, bezogen auf die gesamte Zusammensetzung, enthalten.

Unter Vitamin F werden üblicherweise essentielle Fettsäuren, insbesondere Linolsäure, Linolensäure und Arachidonsäure, verstanden.

Vitamin H ist eine andere Bezeichnung für Biotin oder Vitamin B₇ (siehe oben).

Zu den fettlöslichen Vitaminen der Vitamin K-Gruppe, denen das Grundgerüst des 2-Methyl-1,4-naphthochinons zugrunde liegt, gehören Phyllochinon (Vitamin K₁), Farnochinon oder Menachinon-7 (Vitamin K2) und Menadion (Vitamin K₃). Vitamin K ist bevorzugt in Mengen von 0,0001 bis 1,0 Gew.-%, insbesondere 0,01 bis 0,5 Gew.-%, jeweils bezogen auf die gesamte Zusammensetzung, enthalten.

Vitamin A-palmitat (Retinylpalmitat), Panthenol, Pantolacton, Nicotinsäureamid, Pyridoxin, Pyridoxamin, Pyridoxal, Biotin, Ascorbylpalmitat, -acetat, Mg-Ascorbylphosphat, Na-Ascorbylphosphat, Natrium- und Magnesiumascorbat und die Tocopherolester, besonders Tocopherylacetat, sind erfindungsgemäß besonders bevorzugt.

In einer weiteren bevorzugten Ausführungsform enthalten die erfindungsgemäßen Zusammensetzungen neben den zwingenden Inhaltsstoffen a) bis d) mindestens eine α-Hydroxycarbonsäure, α-Ketocarbonsäure oder β-Hydroxycarbonsäure oder deren Ester-, Lacton- oder Salzform. Erfindungsgemäß geeignete α-Hydroxycarbonsäuren oder α-Ketocarbonsäuren sind Glycolsäure, Milchsäure, Weinsäure, Citronensäure, 2-Hydroxybutansäure, 2,3-Dihydroxypropansäure, 2-Hydroxypentansäure, 2-Hydroxyhexansäure, 2-Hydroxyheptansäure, 2-Hydroxyoctansäure, 2-Hydroxydecansäure, 2-Hydroxydodecansäure, 2-Hydroxytetradecansäure, 2-Hydroxyhexadecansäure, 2-Hydroxyoctadecansäure, Mandelsäure, 4-Hydroxymandelsäure, Äpfelsäure, Erythrarsäure, Threarsäure, Glucarsäure, Galactarsäure, Mannarsäure, Gularsäure, 2-Hydroxy-2-methylbernsteinsäure, Gluconsäure, Brenztraubensäure, Glucuronsäure und Galacturonsäure. Besonders bevorzugte α-Hydroxycarbonsäuren sind Milchsäure, Citronensäure, Glycolsäure und Gluconsäure. Eine besonders bevorzugte β-Hydroxycarbonsäure ist Salicylsäure. Die Ester der genannten Säuren sind ausgewählt aus den Methyl-, Ethyl-, Propyl-, Isopropyl-, Butyl-, Amyl-, Pentyl-, Hexyl-, 2-Ethylhexyl-, Octyl-, Decyl-, Dodecyl- und Hexadecylestern. Die α-Hydroxycarbonsäuren, α-Ketocarbonsäuren oder β-Hydroxycarbonsäuren oder ihre Derivate sind vorzugsweise in Mengen von 0,1 - 10 Gew.-%, bevorzugt 0,5 - 5 Gew.-%, jeweils bezogen auf die gesamte Zusammensetzung, enthalten.

In einer weiteren bevorzugten Ausführungsform enthalten die erfindungsgemäßen Zusammensetzungen neben den zwingenden Inhaltsstoffen a) bis d) mindestens ein Flavonoid oder mindestens einen Flavonoid-reichen Pflanzenextrakt.

Die erfindungsgemäß bevorzugten Flavonoide umfassen die Glycoside der Flavone, der Flavanone, der 3-Hydroxyflavone (Flavonole), der Aurone und der Isoflavone. Besonders bevorzugte Flavonoide sind ausgewählt aus Naringin (Aurantiin, Naringenin-7-rhamnoglucosid), α-Glucosylrutin, α-Glucosylmyricetin, α-Glucosylisoquercetin, α-Glucosylquercetin, Hesperidin (3',5,7-Trihydroxy-4'-methoxyflavanon-7-rhamnoglucosid, Hesperitin-7-O-rhamnoglucosid), Neohesperidin, Rutin (3,3',4',5,7-Pentahydroxyflavon-3-rhamnoglucosid, Quercetin-3-rhamnoglucosid), Troxerutin (3,5-Dihydroxy-3',4',7-tris(2-hydroxyethoxy)-flavon-3-(6-O-(6-deoxy-α-L-mannopyranosyl)-β-D-glucopyranosid)), Monoxerutin (3,3',4',5-Tetrahydroxy-7-(2-hydroxyethoxy)-flavon-3-(6-O-(6-deoxy-α-L-mannopyranosyl)-β-D-glucopyranosid)), Diosmin (3',4',7-Trihydroxy-5-methoxyflavanon-7-rhamnoglucosid), Eriodictin und Apigenin-7-glucosid (4',5,7-Trihydroxyflavon-7-glucosid).

Erfindungsgemäß außerordentlich bevorzugte Flavonoide sind α-Glucosylrutin, Naringin und Apigenin-7-glucosid.

Ebenfalls bevorzugt sind die aus zwei Flavonoideinheiten aufgebauten Biflavonoide, die z. B. in Gingko-Arten vorkommen. Weitere bevorzugte Flavonoide sind die Chalkone, vor allem Phloricin, Hesperidinmethylchalkon und Neohesperidindihydrochalkon.

Erfindungsgemäß werden die Flavonoide vorzugsweise in Mengen von 0,0001 bis 1 Gew.-%, bevorzugt 0,0005 bis 0,5 Gew.-% und besonders bevorzugt 0,001 bis 0,1 Gew.-%, jeweils bezogen auf die Flavonoidaktivsubstanz in der gesamten kosmetischen Zusammensetzung, eingesetzt.

In einer weiteren bevorzugten Ausführungsform enthalten die erfindungsgemäßen Zusammensetzungen neben den zwingenden Inhaltsstoffen a) bis d) mindestens ein Isoflavonoid oder mindestens einen Isoflavonoid-reichen Pflanzenextrakt. Zu den Isoflavonoiden werden an dieser Stelle die Isoflavone und die Isoflavon-Glycoside gezählt.

Unter Isoflavonen sind im Sinne der vorliegenden Erfindung Stoffe zu verstehen, die Hydrierungs-, Oxidations- oder Substitutionsprodukte des 3-Phenyl-4H-1-benzopyrans darstellen, wobei eine Hydrierung in der 2,3-Stellung des Kohlenstoffgerüsts vorliegen kann, eine Oxidation unter Ausbildung einer Carbonylgruppe in der 4-Stellung vorliegen kann, und unter Substitution der Ersatz eines oder mehrerer Wasserstoffatome durch Hydroxy- oder Methoxy-Gruppen zu verstehen ist. Zu den erfindungsgemäß bevorzugten Isoflavonen zählen beispielsweise Daidzein, Genistein, Prunetin, Biochanin, Orobol, Santal, Pratensein, Irigenin, Glycitein, Biochanin A und Formononetin. Als Isoflavone besonders bevorzugt sind Daidzein, Genistein, Glycitein und Formononetin.

In den erfindungsgemäß bevorzugten Isoflavon-Glycosiden sind die Isoflavone über mindestens eine Hydroxygruppe mit mindestens einem Zucker glycosidisch verknüpft. Als Zucker kommen Mono- oder Oligosaccharide, insbesondere D-Glucose, D-Galactose, D-Glucuronsäure, D-Galacturonsäure, D-Xylose, D-Apiose, L-Rhamnose, L-Arabinose und Rutinose in Betracht. Erfindungsgemäß besonders bevorzugte Isoflavon-Glycoside sind Daidzin und Genistin.

Erfindungsgemäß weiterhin bevorzugt ist es, wenn die Isoflavone und/oder deren Glycoside als Bestandteile eines aus einer Pflanze gewonnenen Substanzgemisches, insbesondere eines pflanzlichen Extraktes, in den Zubereitungen enthalten sind. Solche pflanzlichen Substanzgemische können in dem Fachmann geläufiger Weise beispielsweise durch Auspressen oder Extrahieren aus Pflanzen wie Soja, Rotklee oder Kichererbsen gewonnen werden. Besonders bevorzugt werden in den erfindungsgemäßen Zubereitungen Isoflavone oder Isoflavon-Glycoside in Form von aus Soja gewonnenen Extrakten eingesetzt, wie sie beispielsweise unter der Produktbezeichnung Soy Protein Isolate SPI (Protein Technology International, St. Louis) oder Soy Phytochemicals Concentrate SPC (Archer Daniels Midland, Decatur) im Handel erhältlich sind. Ein weiterer besonders bevorzugter Isoflavonoid-reicher Pflanzenextrakt ist Apfelkernextrakt, insbesondere das Handelsprodukt Ederline von Seporga. Ederline enthält Phytohormone, Isoflavonoide, Phytosterole, Triterpenoide, Tocopherole und natürliche Wachse. Erfindungsgemäß werden die Isoflavonoide vorzugsweise in Mengen von 0,00001 bis 1 Gew.-%, bevorzugt 0,0005 bis 0,5 Gew.-% und besonders bevorzugt 0,001 bis 0,1 Gew.-%, jeweils bezogen auf die Isoflavonoidaktivsubstanz in der gesamten kosmetischen Zusammensetzung, eingesetzt.

In einer weiteren bevorzugten Ausführungsform enthalten die erfindungsgemäßen Zusammensetzungen neben den zwingenden Inhaltsstoffen a) bis d) mindestens ein Polyphenol oder einen Polyphenol-reichen Pflanzenextrakt.

Unter Polyphenolen sind erfindungsgemäß aromatische Verbindungen zu verstehen, die mindestens zwei phenolische Hydroxy-Gruppen im Molekül enthalten. Hierzu zählen die drei Dihydroxybenzole Brenzcatechin, Resorcin und Hydrochinon, weiterhin Phloroglucin, Pyrogallol und Hexahydroxybenzol. In der Natur treten freie und veretherte Polyphenole beispielsweise in Blütenfarbstoffen (Anthocyanidine, Flavone), in Gerbstoffen (Catechine, Tannine), als Flechten-oder Farn-Inhaltsstoffe (Usninsäure, Acylpolyphenole), in Ligninen und als Gallussäure-Derivate auf. Bevorzugte Polyphenole sind Flavone, Catechine, Usninsäure, und als Tannine die Derivate der Gallussäure, Digallussäure und Digalloylgallussäure. Besonders bevorzugte Polyphenole sind die monomeren Catechine, das heißt die Derivate der Flavan-3-ole, und Leukoanthocyanidine, das heißt die Derivate der Leukoanthocyanidine, die bevorzugt in 5,7,3',4',5'-Stellung phenolische Hydroxygruppen tragen, bevorzugt Epicatechin und Epigallocatechin, sowie die daraus durch Selbstkondensation entstehenden Gerbstoffe. Solche Gerbstoffe werden bevorzugt nicht in isolierter Reinsubstanz, sondern als Extrakte gerbstoffreicher Pflanzenteile eingesetzt, z. B. Extrakte von Catechu, Quebracho, Eichenrinde und Pinienrinde sowie anderen Baumrinden, Blättern von Grünem Tee (camellia sinensis) und Mate. Ebenfalls besonders bevorzugt sind die Tannine.

Ein besonders bevorzugter Polyphenol-reicher kosmetischer Wirkstoff ist das Handelsprodukt Sepivinol R, ein Extrakt aus Rotwein, erhältlich von der Firma Seppic. Ein weiterer besonders bevorzugter Polyphenol-reicher kosmetischer Wirkstoff ist das Handelsprodukt Crodarom Chardonnay L, ein Extrakt aus den Kernen der Chardonnay-Traube, erhältlich von der Firma Croda.

Erfindungsgemäß werden die Polyphenole vorzugsweise in Mengen von 0,001 bis 10 Gew.-%, bevorzugt 0,005 bis 5 Gew.-% und besonders bevorzugt 0,01 bis 3 Gew.-%, jeweils bezogen auf die gesamte kosmetische Zusammensetzung, eingesetzt.

In einer weiteren bevorzugten Ausführungsform enthalten die erfindungsgemäßen Zusammensetzungen neben den zwingenden Inhaltsstoffen a) bis d) mindestens ein Ubichinon oder ein Ubichinol oder deren Derivate. Ubichinole sind die reduzierte Form der Ubichinone. Die erfindungsgemäß bevorzugten Ubichinone weisen die Formel (II) auf: 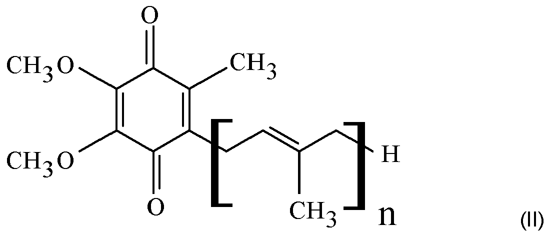 mit n = 6, 7, 8, 9 oder 10.

Besonders bevorzugt ist das Ubichinon der Formel (II) mit n = 10, auch bekannt als Coenzym Q10. Erfindungsgemäß werden die Ubichinone, Ubichinole oder deren Derivate vorzugsweise in Mengen von 0,0001 bis 1 Gew.-%, bevorzugt 0,001 bis 0,5 Gew.-% und besonders bevorzugt 0,005 bis 0,1 Gew.-%, jeweils bezogen auf die gesamte Zusammensetzung, eingesetzt.

In einer weiteren bevorzugten Ausführungsform enthalten die erfindungsgemäßen Zusammensetzungen neben den zwingenden Inhaltsstoffen a) bis d) Silymarin. Silymarin stellt erfindungsgemäß ein früher als einheitliche Substanz angesehenes Wirkstoff-Konzentrat aus den Früchten der Mariendistel (Silybum marianum) dar. Die Hauptbestandteile des Silymarins sind Silybin (Silymarin I), Silychristin (Silymarin II) und Silydianin, die zur Gruppe der Flavanolignane gehören.

Erfindungsgemäß wird Silymarin vorzugsweise in Mengen von 0,0001 bis 1 Gew.-%, bevorzugt 0,001 bis 1,0 Gew.-% und besonders bevorzugt 0,005 bis 0,5 Gew.-%, jeweils bezogen auf die gesamte Zusammensetzung, eingesetzt.

In einer weiteren bevorzugten Ausführungsform enthalten die erfindungsgemäßen Zusammensetzungen neben den zwingenden Inhaltsstoffen a) bis d) mindestens ein natürlich vorkommendes Xanthin-Derivat, ausgewählt aus Coffein, Theophyllin, Theobromin und Aminophyllin.

Erfindungsgemäß sind die natürlich vorkommenden Xanthin-Derivate vorzugsweise in Mengen von 0,0001 bis 1 Gew.-%, bevorzugt 0,001 bis 0,5 Gew.-% und besonders bevorzugt 0,005 bis 0,1 Gew.-%, jeweils bezogen auf die gesamte Zusammensetzung, enthalten.

In einer weiteren bevorzugten Ausführungsform enthalten die erfindungsgemäßen Zusammensetzungen neben den zwingenden Inhaltsstoffen a) bis d) Ectoin. Ectoin ist der Trivialname für 2-Methyl-1,4,5,6-tetrahydropyrimidin-4-carboxylat. Erfindungsgemäß ist Ectoin vorzugsweise in Mengen von 0,0001 bis 1 Gew.-%, bevorzugt 0,001 bis 0,5 Gew.-% und besonders bevorzugt 0,005 bis 0,01 Gew.-%, jeweils bezogen auf die gesamte Zusammensetzung, enthalten.

In einer weiteren bevorzugten Ausführungsform enthalten die erfindungsgemäßen Zusammensetzungen neben den zwingenden Inhaltsstoffen a) bis d) mindestens eine anorganische und/oder mindestens eine organische UV-Filtersubstanz.

Bei den UV-Filtersubstanzen handelt es sich um bei Raumtemperatur flüssig oder kristallin vorliegende Substanzen, die in der Lage sind, ultraviolette Strahlen zu absorbieren und die aufgenommene Energie in Form längerwelliger Strahlung, z. B. Wärme, wieder abzugeben. Man unterscheidet UVA-Filter und UVB-Filter. Die UVA- und UVB-Filter können sowohl einzeln als auch in Mischungen eingesetzt werden. Der Einsatz von Filter-Mischungen ist erfindungsgemäß bevorzugt. Die erfindungsgemäß verwendeten organischen UV-Filter sind ausgewählt aus den physiologisch verträglichen Derivaten von Dibenzoylmethan, Zimtsäureestern, Diphenylacrylsäureestern, Benzophenon, Campher, p-Aminobenzoesäureestern, o-Aminobenzoesäureestern, Salicylsäureestern, Benzimidazolen, symmetrisch oder unsymmetrisch substituierten 1,3,5-Triazinen, monomeren und oligomeren 4,4-Diarylbutadiencarbonsäureestern und -carbonsäureamiden, Ketotricyclo(5.2.1.0)decan, Benzalmalonsäureestern, Benzoxazol sowie beliebigen Mischungen der genannten Komponenten. Die organischen UV-Filter können öllöslich oder wasserlöslich sein. Erfindungsgemäß besonders bevorzugte öllösliche UV-Filter sind 1-(4-tert.-Butylphenyl)-3-(4'-methoxyphenyl)propan-1,3-dion (Parsol^{®} 1789), 1-Phenyl-3-(4'-isopropylphenyl)-propan-1,3-dion, 3-(4'-Methylbenzyliden)-D,L-campher, 4-(Dimethylamino)-benzoesäure-2-ethylhexylester, 4-(Dimethylamino)benzoesäure-2-octylester, 4-(Dimethylamino)-benzoesäureamylester, 4-Methoxyzimtsäure-2-ethylhexylester, 4-Methoxyzimtsäurepropylester, 4-Methoxyzimtsäureisopentylester, 2-Cyano-3,3-phenylzimtsäure-2-ethylhexylester (Octocrylene), Salicylsäure-2-ethylhexylester, Salicylsäure-4-isopropylbenzylester, Salicylsäurehomomenthylester (3,3,5-Trimethyl-cyclohexylsalicylat), 2-Hydroxy-4-methoxybenzophenon, 2-Hydroxy-4-methoxy-4'-methylbenzophenon, 2,2'-Dihydroxy-4-methoxybenzophenon, 4-Methoxybenzmalonsäuredi-2-ethylhexylester, 2,4,6-Trianilino-(p-carbo-2'-ethyl-1'-hexyloxy)-1,3,5-triazin (Octyl Triazone, Uvinul^{®} T 150), Dimethicodiethylbenzal malonate (CAS-Nr. 207574-74-1, Parsol^{®} SLX), Dioctyl Butamido Triazone (Uvasorb^{®} HEB), 2,4-bis-[5-1(di- methylpropyl)benzoxazol-2-yl-(4-phenyl)-imino]-6-(2-ethylhexyl)-imino-1,3,5-triazin (CAS Nr. 288254-16-0, Uvasorb^{®} K2A) und sowie beliebige Mischungen der genannten Komponenten.

Bevorzugte wasserlösliche UV-Filter sind 2-Phenylbenzimidazol-5-sulfonsäure, Phenylen-1,4-bis-(2-benzimidazyl)-3,3'-5,5'-tetrasulfonsäure und deren Alkali-, Erdalkali-, Ammonium-, Alkylammonium-, Alkanolammonium- und Glucammoniumsalze, Sulfonsäurederivate von Benzophenonen, vorzugsweise 2-Hydroxy-4-methoxybenzophenon-5-sulfonsäure und ihre Salze, Sulfonsäurederivate des 3-Benzylidencamphers, wie z. B. 4-(2-Oxo-3-bornylidenmethyl)benzolsulfonsäure und 2-Methyl-5-(2-oxo-3-bornyliden)sulfonsäure und deren Salze.

Einige der öllöslichen UV-Filter können selbst als Lösungsmittel oder Lösungsvermittler für andere UV-Filter dienen. So lassen sich beispielsweise Lösungen des UV-A-Filters 1-(4-tert.-Butylphenyl)-3-(4'methoxyphenyl)propan-1,3-dion (z. B. Parsol^{®} 1789) in verschiedenen UV-B-Filtern herstellen.

Die erfindungsgemäßen Zusammensetzungen enthalten daher in einer weiteren bevorzugten Ausführungsform 1-(4-tert.-Butylphenyl)-3-(4'-methoxyphenyl)propan-1,3-dion in Kombination mit mindestens einem UV-B-Filter, ausgewählt aus 4-Methoxyzimtsäure-2-ethylhexylester, 2-Cyano-3,3-phenylzimtsäure-2-ethylhexylester, Salicylsäure-2-ethylhexylester und 3,3,5-Trimethyl-cyclohexylsalicylat. In diesen Kombinationen liegt das Gewichtsverhältnis von UV-B-Filter zu 1-(4-tert.-Butylphenyl)-3-(4'methoxyphenyl)propan-1,3-dion zwischen 1:1 und 10:1, bevorzugt zwischen 2:1 und 8:1, das molare Verhältnis liegt entsprechend zwischen 0,3 und 3,8, bevorzugt zwischen 0,7 und 3,0.

Bei den erfindungsgemäß bevorzugten anorganischen Lichtschutzpigmenten handelt es sich um feindisperse oder kolloiddisperse Metalloxide und Metallsalze, beispielsweise Titandioxid, Zinkoxid, Eisenoxid, Aluminiumoxid, Ceroxid, Zirkoniumoxid, Silicate (Talk) und Bariumsulfat. Die Partikel sollten dabei einen mittleren Durchmesser von weniger als 100 nm, vorzugsweise zwischen 5 und 50 nm und insbesondere zwischen 15 und 30 nm aufweisen, so genannte Nanopigmente. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z. B. Titandioxid T 805 (Degussa) oder Eusolex^{®} T2000 (Merck). Als hydrophobe Coatingmittel kommen dabei vor allem Silicone und dabei speziell Trialkoxyoctylsilane oder Simethicone in Frage. Besonders bevorzugt sind Titandioxid und Zinkoxid.

Erfindungsgemäß sind die organischen UV-Filtersubstanzen in Mengen von 0,1 - 30 Gew.-%, bevorzugt 0,5 - 20 Gew.-%, besonders bevorzugt 1,0 - 15 Gew.-% und außerordentlich bevorzugt 3,0 - 10 Gew.-%, jeweils bezogen auf die gesamte Zusammensetzung, enthalten. Erfindungsgemäß sind die anorganischen UV-Filtersubstanzen vorzugsweise in Mengen von 0,1 - 15 Gew.-%, bevorzugt 0,5 - 10 Gew.-%, besonders bevorzugt 1,0 - 5 Gew.-% und außerordentlich bevorzugt 2,0 - 4,0 Gew.-%, jeweils bezogen auf die gesamte Zusammensetzung, enthalten.

In einer weiteren bevorzugten Ausführungsform enthalten die erfindungsgemäßen Zusammensetzungen neben den zwingenden Inhaltsstoffen a) bis d) mindestens einen selbstbräunenden Wirkstoff. Erfindungsgemäß bevorzugte selbstbräunende Wirkstoffe sind ausgewählt aus Dihydroxyaceton und Erythrulose.

Erfindungsgemäß sind die selbstbräunenden Wirkstoffe vorzugsweise in Mengen von 0,1 - 15 Gew.-%, bevorzugt 0,5 - 10 Gew.-%, besonders bevorzugt 1,0 - 5 Gew.-% und außerordentlich bevorzugt 2,0 - 4,0 Gew.-%, jeweils bezogen auf die gesamte Zusammensetzung, enthalten.

In einer weiteren bevorzugten Ausführungsform enthalten die erfindungsgemäßen Zusammensetzungen neben den zwingenden Inhaltsstoffen a) bis d) mindestens einen hautaufhellenden Wirkstoff. Erfindungsgemäß bevorzugte hautaufhellende Wirkstoffe sind ausgewählt aus Ascorbinsäure, den Estern der Ascorbinsäure mit Phosphorsäure und/oder organischen C₂-C₂₀-Carbonsäuren sowie deren Alkali- und Erdalkalimetallsalzen, Kojisäure, Hydrochinon, Arbutin, Maulbeerbaumextrakt und Süßholzextrakt sowie Mischungen hiervon. Sowohl als Einzelsubstanz wie auch in Mischung bevorzugt sind die Ascorbinsäurederivate sowie Kojisäure bevorzugt. Besonders bevorzugt sind Natriumascorbylphosphat, Magnesiumascorbylphosphat, Ascorbylmonopalmitat, Ascorbyldipalmitat, Ascorbylmonostearat, Ascorbyldistearat, Ascorbylmonoethylhexanoat, Ascorbyldiethylhexanoat, Ascorbylmonooctanoat, Ascorbyldioctanoat, Ascorbylmonoisostearat und Ascorbyldiisostearat. Die erfindungsgemäß außerordentlich bevorzugten Ascorbinsäurederivate sind Natriumascorbylphosphat und Magnesiumascorbylphosphat. Die hautaufhellenden Wirkstoffe sind vorzugsweise in einer Menge von 0,05 bis 5 Gew.-%, bevorzugt von 0,1 - 2 Gew-%, jeweils bezogen auf die gesamte Zusammensetzung, enthalten.

In einer weiteren bevorzugten Ausführungsform enthalten die erfindungsgemäßen Zusammensetzungen neben den zwingenden Inhaltsstoffen a) bis d) mindestens einen hautberuhigenden Wirkstoff. Erfindungsgemäß bevorzugte hautberuhigende Wirkstoffe sind ausgewählt aus Allantoin, α-Bisabolol und α-Liponsäure.

Die hautberuhigenden Wirkstoffe sind vorzugsweise in Mengen von 0,001 bis 5 Gew.-%, bevorzugt 0,01 bis 2 Gew.-% und besonders bevorzugt 0,1 bis 1 Gew.-%, jeweils bezogen auf die gesamte Zusammensetzung, enthalten.

In einer weiteren bevorzugten Ausführungsform enthalten die erfindungsgemäßen Zusammensetzungen neben den zwingenden Inhaltsstoffen a) bis d) mindestens einen sebumregulierenden Wirkstoff. Erfindungsgemäß bevorzugte sebumregulierende Wirkstoffe sind ausgewählt aus Acnacidol, Azelainsäure, Azeloglycina und einem Extrakt aus Spiraea Ulmaria. Der Extrakt ist z.B. im Produkt Seburegul der Firma Silab enthalten. Die sebumregulierenden Wirkstoffe sind vorzugsweise in Mengen von 0,001 bis 5 Gew.-%, bevorzugt 0,01 bis 2 Gew.-% und besonders bevorzugt 0,1 bis 1 Gew.-%, jeweils bezogen auf die gesamte Zusammensetzung, enthalten.

In einer weiteren bevorzugten Ausführungsform enthalten die erfindungsgemäßen Zusammensetzungen neben den zwingenden Inhaltsstoffen a) bis d) mindestens einen Desoxyzucker oder ein mindestens einen Desoxyzucker-Baustein enthaltendes Polysaccharid. Erfindungsgemäß bevorzugte Desoxyzucker sind ausgewählt aus Rhamnose und Fucose. Ein erfindungsgemäß besonders bevorzugtes, Desoxyzucker-Bausteine enthaltendes Polysaccharid ist das Handelsprodukt Fucogel^{®}.

Die Desoxyzucker oder Desoxyzucker-Bausteine enthaltenden Polysaccharide sind vorzugsweise in Mengen von 0,001 bis 5 Gew.-%, bevorzugt 0,01 bis 2 Gew.-% und besonders bevorzugt 0,1 bis 1 Gew.-%, jeweils bezogen auf die gesamte Zusammensetzung, enthalten.

Ein weiterer Gegenstand der vorliegenden Erfindung ist die Verwendung von Mischungen aus
a) mindestens einem Ester ausgewählt aus einer oder mehrerer Verbindungen der Gruppe, die gebildet wird aus Linolsäureestern, Linolensäurestern und Arachidonsäureestern;;
b) Panthenol;
c) Tocopherylacetat;
d) N,N'-Bis-(2-hydroxyethyl)harnstoff
in Hautbehandlungsmitteln zur Erhöhung des Feuchtigkeitsgehaltes und/oder zur Erhöhung des ATP-Gehaltes der Haut.

Bezüglich bevorzugter erfindungsgemäßer Verwendungen gilt mutatis mutandis das zu den Inhaltsstoffen der erfindungsgemäßen Mitteln Gesagte.

### Beispiele:

Die erfindungsgemäßen Zusammensetzungen wurden gegen nicht-erfindungsgemäße Zusammensetzungen mit dem Phenion-Hautmodell verglichen.

Das Phenion® full thickness skin model (Phenion FT-Modell) ist ein dreidimensionales, lebendes mehrschichtiges Vollhautmodell, welches aus Keratinozyten und Fibroblasten desselben menschlichen Donors aufgebaut ist.

Die Keratinozyten und Fibroblasten werden aus gesundem Vorhautgewebe gewonnen. In einem ersten Schritt werden die vereinzelten Fibroblasten in eine Kollagenmatrix eingebettet, welche die Ausbildung der dermalen Struktur unterstützt. Nach ca. 2 Wochen werden in einem zweiten Schritt auf diese rekonstruierte Dermis die Keratinozyten ausgesät. Nach einigen Tagen ist eine Ausdifferenzierung und Schichtung der Keratinozyten erkennbar.

Für eine natürliche Ausdifferenzierung ist auch im Phenion FT-Modell der Luftkontakt zum richtigen Zeitpunkt essentiell. Nach insgesamt 5-wöchiger Kultivierung, wovon die letzten 12 Tage an der Luft-Medium-Grenze, ist die Hautstruktur analog zu nativer Haut vollständig ausgebildet. Die Epidermis umfasst 8 bis 10 Zellschichten, weist die epidermistypische Schichtung auf (Stratum basale, Stratum spinosum, Stratum granulosum und Stratum corneum) und ist mit der darunter liegenden rekonstruierten Dermis fest verhaftet. Mit zunehmendem Alter findet eine Verdickung des Stratum corneum und damit eine Verhornung statt.

Die Haumodelle wurden topisch mit 5 µl der jeweiligen Zusammensetzung behandelt, wobei pro Testprodukt jeweils 3 Hautmodelle behandelt durchgeführt wurden. Nach 24 Stunden wurden je zwei Hautmodelle schockgefroren, um den ATP-Gehalt zu bestimmen. Das jeweils dritte Hautmodell wurde in Paraffin eingebettet und 48 Stunden nach der Behandlung mit Hematoxin-Eosin angefärbt. Die Studien wurden zweifach mit Hautmodellen unterschiedlicher Batches durchgeführt.

### Quantifizierung von ATP:

Adenosin-5'-Triphoshat (ATP) ist ein Marker für die Zellviabilität, weil es in allen metabolisch aktiven Zellen enthalten ist und seine Konzentration sehr schnell abnimmt, wenn die Zellen Nekrose oder Apoptose erleiden.

Der ATP-Gehalt wurde mit Hilfe des ATP lite 1 step assay Systems bestimmt, das auf der Emission von Licht bei der Rreaktion von ATP mit zugefügter Luciferase und D-Luciferin beruht. Die Hautmodelle wurden dazu in 1 ml Tris-EDTA-Puffer überführt und mittels eines Qiagen Homegenisators (5 min / 30 Hz) disruptiert. Nach Inkubation bei 110°C für 5 min und Zentrfugation bei 13000 rpm für eine Minute bei einer temperatur von 4°C, wurden das Testmedium und Luciferase Reagens auf eine schwarze Platte aufgebracht und die Biolumineszenz gemessen.

Es wurden folgende Zusammensetzungen untersucht (alle Angaben in Gew.-%):

| | **E** | **V** |
|---|---|---|
| Emulsiphos 677660 | 0,5 | 0,5 |
| Cetearyl Alcohol | 1,0 | 1,0 |
| Cutina MD | 0,5 | 0,5 |
| Cetiol A | 5,0 | 5,0 |
| 2-Ethylhexylpalmitat | 2,0 | 2,0 |
| Cetiol SB 45 | 0,5 | 0,5 |
| NOVATA AB PH | 0,5 | 0,5 |
| Glycerin, 99,5% | 5,0 | 5,0 |
| Sorbitol 70% HS DAB | 3,0 | 3,0 |
| Tego Carbomer 140 | 0,3 | 0,3 |
| Natriumhydroxid Perlen | 0,04 | 0,04 |
| Aluminumstärkeoctenylsuccinat | 1,0 | 1,0 |
| Vitamin F Glycerinester | 0,3 | - |
| D-Panthenol | 0,1 | - |
| Vitamin E Acetat | 0,05 | - |
| N,N'-Bis-(2-hydroxyethyl)harnstoff | 3,0 | - |
| Phenonip ME | 0,5 | 0,5 |
| Simulgel EPG | 0,5 | 0,5 |
| Parfum | 0,3 | 0,3 |
| Wasser, vollentsalzt | ad 100 | ad 100 |
| Emulsiphos^{®} 677660 | INCI-Bezeichnung: Potassium Cetyl Phosphate, Hydrogenated Palm Glycerides (Symrise) | |
| Cutina^{®} MD | Glycerinmono/distearat (INCI-Bezeichnung: Glyceryl Stearate) (Cognis) | |
| Cetiol^{®} A | Laurinsäurehexylester (INCI-Bezeichnung: Hexyl Laurate) (Cognis) | |
| Cetiol^{®} SB | INCI-Bezeichnung: Shea Butter Butyrospermum Parkii (Linne) (Cognis) | |
| Novata^{®} AB | INCI-Bezeichnung: Coco Glycerides (Cognis) | |
| Tego Carbomer^{®} 140 | Carboxyvinylpolymer (INCI-Bezeichnung: Carbomer) (Goldschmidt) | |
| Phenonip^{®} | Hydroxybenzoesäuremethylester-Hydroxybenzoe-säureethylester-Hydroxybenzoesäurepropylester-Hydroxybenzoesäurebutylester-Phenoxyethanol-Gemisch (ca. 28 % Aktivsubstanz; INCI-Bezeichnung: Phenoxyethanol, Methylparaben, Ethylparaben, Propylparaben, Butylparaben) (Nipa) | |
| Simulgel^{®} EPG | INCI-Bezeichnung: SODIUM ACRYLATE/SODIUM ACRYLOYLDIMETHYL TAURATE COPOLYMER, POLYISOBUTENE, CAPRYLYL/CAPRYL GLUCOSIDE (Seppic) | |

| | | |
|---|---|---|
| Nach 24 Stunden Inkubation wurde der ATP-Gehalt bestimmt, wobei die Vergleichsprobe als 100% normiert wurde. Die erfindungsgemäße Zusammensetzung erreichte einen Wert von 118%. Bei der ATP-Bestimmung aus der zweiten Probe, wobei die Vergleichsprobe wiederum als 100% normiert wurde, erreichte die erfindungsgemäße Zusammensetzung einen Wert von 133%. | | |

## Patentansprüche

1. Kosmetisches oder dermatologisches Mittel, enthaltend - bezogen auf sein Gewicht -
a) 0,1 bis 0,5 Gew.-% mindestens eines Esters einer aliphatischen, mehrfach ungesättigten (C₁₆ bis C₃₀)-Carbonsäure,
b) 0,05 bis 0,3 Gew.-% Panthenol;
c) 0,01 bis 0,1 Tocopherylacetat;
d) 0,5 bis 5 Gew.-% mindestens eines Harnstoff-Derivats der Formel (I) 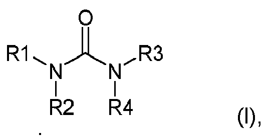 worin
die Reste R1, R2, R3 und R4 unabhängig voneinander für ein Wasserstoffatom, eine C₁-C₄-Alkylgruppe, eine C₂-C₆-Alkenylgruppe oder eine C₂-C₆-Hydroxyalkylgruppe mit mindestens einer Hydroxygruppe steht, mit der Maßgabe, dass mindestens einer der Reste R1, R2, R3 und R4 für eine C₂-C₆-Hydroxyalkylgruppe mit mindestens einer Hydroxygruppe steht.

2. Kosmetisches oder dermatologisches Mittel nach Anspruch 1, **dadurch gekennzeichnet, daß** der Esters einer aliphatischen, mehrfach ungesättigten (C₁₆ bis C₃₀)-Carbonsäure ausgewählt wird aus einer oder mehrerer Verbindungen der Gruppe, die gebildet wird aus Linolsäureestern, Linolensäurestern und Arachidonsäureestern.

3. Kosmetisches oder dermatologisches Mittel nach Anspruch 1, **dadurch gekennzeichnet, daß** es 0,15 bis 0,45 Gew.-%, vorzugsweise 0,2 bis 0,4 Gew.-%, weiter bevorzugt 0,25 bis 0,35 Gew.-% und insbesondere 0,28 bis 0,32 Gew.-% mindestens eines Esters einer aliphatischen, mehrfach ungesättigten (C₁₆ bis C₃₀)-Carbonsäure enthält.

4. Kosmetisches oder dermatologisches Mittel nach Anspruch 1, **dadurch gekennzeichnet, daß** es 0,15 bis 0,45 Gew.-%, vorzugsweise 0,2 bis 0,4 Gew.-%, weiter bevorzugt 0,25 bis 0,35 Gew.-% und insbesondere 0,28 bis 0,32 Gew.-% mindestens eines Esters aus der Gruppe, die gebildet wird aus Linolsäureestern, Linolensäurestern und Arachidonsäureestern, enthält.

5. Kosmetisches oder dermatologisches Mittel nach einem der Ansprüche 1 oder 2, **dadurch gekennzeichnet, daß** es als Ester einer aliphatischen, mehrfach ungesättigten (C₁₆ bis C₃₀)-Carbonsäure 0,15 bis 0,45 Gew.-%, vorzugsweise 0,2 bis 0,4 Gew.-%, weiter bevorzugt 0,25 bis 0,35 Gew.-% und insbesondere 0,28 bis 0,32 Gew.-% mindestens eines Esters aus der Gruppe, die gebildet wird aus Linolsäureethylester, Linolensäurethylester und Arachidonsäureethylester, enthält.

6. Kosmetisches oder dermatologisches Mittel nach einem der Ansprüche 1 oder 2, **dadurch gekennzeichnet, daß** es als Ester einer aliphatischen, mehrfach ungesättigten (C₁₆ bis C₃₀)-Carbonsäure 0,15 bis 0,45 Gew.-%, vorzugsweise 0,2 bis 0,4 Gew.-%, weiter bevorzugt 0,25 bis 0,35 Gew.-% und insbesondere 0,28 bis 0,32 Gew.-% mindestens eines Esters aus der Gruppe, die gebildet wird aus Linolsäureglycerylestern, Linolensäureglycerylstern und Arachidonsäureglycerylestern, enthält.

7. Kosmetisches oder dermatologisches Mittel nach einem der Ansprüche 1 oder 2, **dadurch gekennzeichnet, daß** es als Ester einer aliphatischen, mehrfach ungesättigten (C₁₆ bis C₃₀-Carbonsäure 0,15 bis 0,45 Gew.-%, vorzugsweise 0,2 bis 0,4 Gew.-%, weiter bevorzugt 0,25 bis 0,35 Gew.-% und insbesondere 0,28 bis 0,32 Gew.-% mindestens eines Esters der Formel (E1) 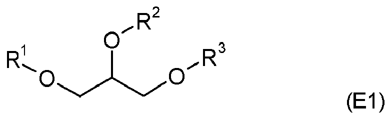 worin R¹, R² und R³ stehen unabhängig voneinander für eine aliphatische, mehrfach ungesättigte (C₁₆ bis C₃₀)-Acylgruppe, insbesondere für dieselbe aliphatische, mehrfach ungesättigte (C₁₆ bis C₃₀)-Acylgruppe, enthält.

8. Kosmetisches oder dermatologisches Mittel nach Anspruch 7, **dadurch gekennzeichnet, daß** R¹, R² und R³ der Formel (E1) ausgewählt werden aus Arachidonoyl (5Z,8Z,11Z,14Z-Eicosatetraenoyl), Linolenoyl (9Z,12Z,15Z-Octadecatrienoyl), Linoloyl (9Z,12Z-Octadecadienoyl).

9. Kosmetisches oder dermatologisches Mittel nach einem der Ansprüche 1 oder 2, **dadurch gekennzeichnet, daß** es als Ester einer aliphatischen, mehrfach ungesättigten (C₁₆ bis C₃₀)-Carbonsäure 0,15 bis 0,45 Gew.-%, vorzugsweise 0,2 bis 0,4 Gew.-%, weiter bevorzugt 0,25 bis 0,35 Gew.-% und insbesondere 0,28 bis 0,32 Gew.-% mindestens eines Esters ausgewählt aus der Gruppe, die gebildet wird aus Glyceryltrilinolenat (INCI-Bezeichnung: Trilinolenin), Glyceryltrilinoleat (INCI-Bezeichnung: Trilinolein), Triarachidoylglyzerin (INCI-Bezeichnung: Triarachidin), enthält.

10. Kosmetisches oder dermatologisches Mittel nach einem der Ansprüche 1 bis 9, **dadurch gekennzeichnet, daß** es 0,06 bis 0,25 Gew.-%, vorzugsweise 0,07 bis 0,2 Gew.-%, weiter bevorzugt 0,08 bis 0,15 Gew.-% und insbesondere 0,09 bis 0,12 Gew.-% Panthenol enthält.

11. Kosmetisches oder dermatologisches Mittel nach einem der Ansprüche 1 bis 10, **dadurch gekennzeichnet, daß** es 0,02 bis 0,09 Gew.-%, vorzugsweise 0,03 bis 0,08 Gew.-%, weiter bevorzugt 0,04 bis 0,07 Gew.-% und insbesondere 0,05 bis 0,06 Gew.-% Tocopherylacetat enthält.

12. Kosmetisches oder dermatologisches Mittel nach einem der Ansprüche 1 bis 11, **dadurch gekennzeichnet, daß** das Harnstoff-Derivat ausgewählt wird aus mindestens einer Verbindung der Gruppe, die gebildet wird aus N-(2-Hydroxyethyl)harnstoff, N,N-Bis-(2-hydroxyethyl)harnstoff, N,N'-Bis-(2-hydroxyethyl)harnstoff, N-(3-Hydroxypropyl)harnstoff, N,N-Bis-(3-hydroxypropyl)harnstoff, N,N'-Bis-(3-hydroxypropyl)harnstoff, N-(2-Hydroxypropyl)harnstoff, N,N-Bis-(2-hydroxypropyl)harnstoff, N,N'-Bis-(2-hydroxypropyl)harnstoff, N-(2-Hydroxy-2-methyl-prop-2-yl)harnstoff, N,N-Bis-(2-hydroxy-2-methyl-prop-2-yl)harnstoff, N,N'-Bis-(2-hydroxy-2-methyl-prop-2-yl)harnstoff, N-(1,3-Dihydroxy-2-methyl-prop-2-yl)harnstoff, N,N-Bis-(1,3-dihydroxy-2-methyl-prop-2-yl)harnstoff, N,N'-Bis-(1,3-dihydroxy-2-methyl-prop-2-yl)harnstoff, N-(1,3-Dihydroxy-2-hydroxymethyl-prop-2-yl)harnstoff, N,N-Bis-(1,3-dihydroxy-2-hydroxymethyl-prop-2-yl)harnstoff und N,N'-Bis-(1,3-dihydroxy-2-hydroxymethyl-prop-2-yl)harnstoff.

13. Kosmetisches oder dermatologisches Mittel nach einem der Ansprüche 1 bis 12, **dadurch gekennzeichnet, daß** es 0,75 bis 4,5 Gew.-%, vorzugsweise 1 bis 4 Gew.-%, weiter bevorzugt 2 bis 3,5 Gew.-% und insbesondere 2,5 bis 3,2 Gew.-% Harnstoff-Derivat(e) der Formel (I) enthält.

14. Kosmetisches oder dermatologisches Mittel nach einem der Ansprüche 1 bis 13, **dadurch gekennzeichnet, daß** es 0,75 bis 4,5 Gew.-%, vorzugsweise 1 bis 4 Gew.-%, weiter bevorzugt 2 bis 3,5 Gew.-% und insbesondere 2,5 bis 3,2 Gew.-% N,N'-Bis-(2-hydroxyethyl)harnstoff enthält.

15. Verwendung von Mischungen aus
a) mindestens einem Ester ausgewählt aus einer oder mehrerer Verbindungen der Gruppe, die gebildet wird aus Linolsäureestern, Linolensäurestern und Arachidonsäureestern;
b) Panthenol;
c) Tocopherylacetat;
d) N,N'-Bis-(2-hydroxyethyl)harnstoff
in Hautbehandlungsmitteln zur Erhöhung des Feuchtigkeitsgehaltes und/oder zur Erhöhung des ATP-Gehaltes der Haut.
